# EUROPEAN PATENT SPECIFICATION

(11) **EP 0 662 003 B1**
(45) Date of publication and mention of the grant of the patent: **29.01.2003**
(21) Application number: 93921230.4
(22) Date of filing: 27.08.1993
(51) Int. Cl.: C12N 15/11, A61K 31/70, C07H 21/04

(54) **OLIGONUCLEOTIDE MODULATION OF CELL ADHESION**
BEEINFLUSSING DER ZELLADHÄSIE DURCH OLIGONUKLEOTIDE
MODULATION OLIGONUCLEOTIDIQUE DE L'ADHESION CELLULAIRE

(30) Priority: 02.09.1992 US 939855; 21.01.1993 US 7997; 17.05.1993 US 63167
(43) Date of publication of application: 12.07.1995
(73) Proprietor: ISIS PHARMACEUTICALS, INC., Carlsbad, CA 92008 (US)
(72) Inventor: BENNET, C. Frank, Carlsbad, CA 92008 (US); MIRABELLI, Christopher K., Dover, MA 02030 (US)
(74) Representative: Hallybone, Huw George
(86) International application number: US9308101
(87) International publication number: WO94005333

(56) References cited:
- WO-A-90/13300
- WO-A-92/03139
- WO-A-94/22488
- PROCEEDINGS OF THE NATIONAL ACADEMY OF SCIENCES OF THE UNITED STATES OF AMERICA, (1989 JAN) 86 (2) 641-4., XP002028518 JOHNSON, J. ET AL.: "De novo expression of intercellular-adhesion molecule 1 in melanoma correlates with increased risk of metastasis"
- GASTROENTEROLOGY, (1991 JAN) 100 (1) 150-9., XP000647856 MALIZIA G ET AL: "Expression of leukocyte adhesion molecules by mucosal mononuclear phagocytes in inflammatory bowel disease."
- PROC ANNU MEET AM ASSOC CANCER RES, (MARCH 1993). VOL. 34, PP. 64, ABSTRACT 381, XP002028519 MIELE, M. ET AL.: "Enhancement of human melanoma metastasis by TNF-alpha is inhibited by antisense ICAM -1 oligonucleotides." & 84TH ANN. MEET. AM. ASSOC. CANCER RES., MAY 19-22, 1993; ORLANDO, FLORIDA,
- JOURNAL OF CELLULAR BIOCHEMISTRY, vol. 17A, 9 - 31 January 1993, page 354 XP000647853 BENNETT, C. ET AL.: "Inhibition of ICAM-1, VCAM-1 and E-selectin expression with antisense oligonucleotides"
- EXPERIMENTAL CELL RESEARCH, (1994 SEP) 214 (1) 231-41., XP000647841 MIELE M E ET AL: "Enhanced metastatic ability of TNF-alpha-treated malignant melanoma cells is reduced by intercellular adhesion molecule -1 ( ICAM -1, CD54 ) antisense oligonucleotides."
- Chemical Reviews, Volume 90, No. 4, issued June 1990, UHLMANN et al., "Antisense Oligonucleotides: A New Therapeutic Principle", pages 544-584, see entire article.
- Journal of Biological Chemistry, Volume 266, No. 27, issued 25 September 1991, CHIANG et al., "Antisense Oligonucleotides Inhibit Intercellular Adhesion Molecule 1 Expression by Two Distinct Mechanisms", pages 18162-18171, see entire article.
- Cell, Volume 59, issued 22 December 1989, OSBORN et al., "Direct Expression Cloning of Vascular Cell Adhesion Molecule 1, a Cytokine-Induced Endothelial Protein that Binds to Lymphocytes", pages 1203-1211, see entire article.
- Science, Volume 246, issued 08 December 1989, RICE et al., "An Inducible Endothelial Cell Surface Glycoprotein Mediates Melanoma Adhesion", pages 1303-1306, see entire article.
- Science, Volume 243, issued 03 March 1989, BEVILACQUA et al., "Endothelial Leukocyte Adhesion Molecule 1: An Inducible Receptor for Neutrophils Related to Complement Regulatory Proteins and Lectins", pages 1160-1165, see entire article.

### SEQUENCE LISTING

(1) GENERAL INFORMATION:
   (i) APPLICANT: Bennett and Mirabelli
   (ii) TITLE OF INVENTION: Oligonucleotide Modulation of Cell Adhesion
   (iii) NUMBER OF SEQUENCES: 85
   (iv) CORRESPONDENCE ADDRESS:
      (A) ADDRESSEE: Woodland Falls Corporate Park
      (B) STREET: 210 Lake Drive East, Suie 201
      (C) CITY: Cherry Hill
      (D) STATE: NJ
      (E) COUNTRY: USA
      (F) ZIP: 08002
   (v) COMPUTER READABLE FORM:
      (A) MEDIUM TYPE: DISKETTE, 3.5 INCH, 1.44 Mb STORAGE
      (B) COMPUTER: IBM PS/2
      (C) OPERATING SYSTEM: PC-DOS
      (D) SOFTWARE: WORDPERFECT 5.0
   (vi) CURRENT APPLICATION DATA:
      (A) APPLICATION NUMBER: n/a
      (B) FILING DATE: Herewith
      (C) CLASSIFICATION:
   (vii) PRIOR APPLICATION DATA:
      (A) APPLICATION NUMBER: 939,855
      (B) FILING DATE: September 2, 1992
   (vii) PRIOR APPLICATION DATA:
      (A) APPLICATION NUMBER: PCT/US91/05209
      (B) FILING DATE: July 23, 1991
   (vii) PRIOR APPLICATION DATA:
      (A) APPLICATION NUMBER: 567,286
      (B) FILING DATE: August 14, 1990
   (vii) PRIOR APPLICATION DATA:
      (A) APPLICATION NUMBER: 007,997
      (B) FILING DATE: January 20, 1993
   (viii) ATTORNEY/AGENT INFORMATION:
      (A) NAME: Jane Massey Licata
      (B) REGISTRATION NUMBER: 32,257
      (C) REFERENCE/DOCKET NUMBER: ISPH-0002
   (ix) TELECOMMUNICATION INFORMATION:
      (A) TELEPHONE: (609) 779-2400
      (B) TELEFAX: (609) 779-8488
(2) INFORMATION FOR SEQ ID NO:1:
   (i) SEQUENCE CHARACTERISTICS:
      (A) LENGTH: 18
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 1:
   TGGGAGCCAT AGCGAGGC 18
(2) INFORMATION FOR SEQ ID NO: 2:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 2:
   GAGGAGCTCA GCGTCGACTG 20
(2) INFORMATION FOR SEQ ID NO: 3:
   (i) SEQUENCE CHARACTERISTICS:
      (A) LENGTH: 21
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 3:
   GACACTCAAT AAATAGCTGG T 21
(2) INFORMATION FOR SEQ ID NO: 4:
   (i) SEQUENCE CHARACTERISTICS:
      (A) LENGTH: 18
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 4:
   GAGGCTGAGG TGGGAGGA 18
(2) INFORMATION FOR SEQ ID NO: 5:
   (i) SEQUENCE CHARACTERISTICS:
      (A) LENGTH: 18
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 5:
   CGATGGGCAG TGGGAAAG 18
(2) INFORMATION FOR SEQ ID NO: 6:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 6:
   GGGCGCGTGA TCCTTATAGC 20
(2) INFORMATION FOR SEQ ID NO: 7:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 7:
   CATAGCGAGG CTGAGGTTGC 20
(2) INFORMATION FOR SEQ ID NO: 8:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 8:
   CGGGGGCTGC TGGGAGCCAT 20
(2) INFORMATION FOR SEQ ID NO: 9:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 9:
   AGAGCCCCGA GCAGGACCAG 20
(2) INFORMATION FOR SEQ ID NO: 10:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 10:
   TGCCCATCAG GGCAGTTTGA 20
(2) INFORMATION FOR SEQ ID NO: 11:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 11:
   GGTCACACTG ACTGAGGCCT 20
(2) INFORMATION FOR SEQ ID NO: 12:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 12:
   CTCGCGGGTG ACCTCCCCTT 20
(2) INFORMATION FOR SEQ ID NO: 13:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 13:
   TCAGGGAGGC GTGGCTTGTG 20
(2) INFORMATION FOR SEQ ID NO: 14:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 14:
(2) INFORMATION FOR SEQ ID NO: 15:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 15:
   CCCCCACCAC TTCCCCTCTC 20
(2) INFORMATION FOR SEQ ID NO: 16:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 16:
   TTGAGAAAGC TTTATTAACT 20
(2) INFORMATION FOR SEQ ID NO: 17:
   (i) SEQUENCE CHARACTERISTICS:
      (A) LENGTH: 14
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 17:
   AGCCATAGCG AGGC 14
(2) INFORMATION FOR SEQ ID NO: 18:
   (i) SEQUENCE CHARACTERISTICS:
      (A) LENGTH: 12
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 18:
   CCATAGCGAG GC 12
(2) INFORMATION FOR SEQ ID NO: 19:
   (i) SEQUENCE CHARACTERISTICS:
      (A) LENGTH: 10
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 19:
   ATAGCGAGGC 10
(2) INFORMATION FOR SEQ ID NO: 20:
   (i) SEQUENCE CHARACTERISTICS:
      (A) LENGTH: 16
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 20:
   TGGGAGCCAT AGCGAG 16
(2) INFORMATION FOR SEQ ID NO: 21:
   (i) SEQUENCE CHARACTERISTICS:
      (A) LENGTH: 16
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 21:
   GGAGCCATAG CGAGGC 16
(2) INFORMATION FOR SEQ ID NO: 22:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 22:
   GCCCAAGCTG GCATCCGTCA 20
(2) INFORMATION FOR SEQ ID NO: 23:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 23:
   TCTGTAAGTC TGTGGGCCTC 20
(2) INFORMATION FOR SEQ ID NO: 24:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 24:
   AGTCTTGCTC CTTCCTCTTG 20
(2) INFORMATION FOR SEQ ID NO: 25:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 25:
   CTCATCAGGC TAGACTTTAA 20
(2) INFORMATION FOR SEQ ID NO: 26:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 26:
   TGTCCTCATG GTGGGGCTAT 20
(2) INFORMATION FOR SEQ ID NO: 27:
   (i) SEQUENCE CHARACTERISTICS:
      (A) LENGTH: 22
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 27:
   TCTGAGTAGC AGAGGAGCTC GA 22
(2) INFORMATION FOR SEQ ID NO: 28:
   (i) SEQUENCE CHARACTERISTICS:
      (A) LENGTH: 22
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 28:
   CAATCATGAC TTCAAGAGTT CT 22
(2) INFORMATION FOR SEQ ID NO: 29:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 29:
   ACCACACTGG TATTTCACAC 20
(2) INFORMATION FOR SEQ ID NO: 30:
   (i) SEQUENCE CHARACTERISTICS:
      (A) LENGTH: 21
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 30:
   GTATGGAAGA TTATAATATA T 21
(2) INFORMATION FOR SEQ ID NO: 31:
   (i) SEQUENCE CHARACTERISTICS:
      (A) LENGTH: 21
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 31:
   CACAATCCTT AAGAACTCTT T 21
(2) INFORMATION FOR SEQ ID NO: 32:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 32:
   ACCTCTGCTG TTCTGATCCT 20
(2) INFORMATION FOR SEQ ID NO: 33:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 33:
   CTGCTGCCTC TGTCTCAGGT 20
(2) INFORMATION FOR SEQ ID NO: 34:
   (i) SEQUENCE CHARACTERISTICS:
      (A) LENGTH: 15
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 34:
   GGTATTTGAC ACAGC 15
(2) INFORMATION FOR SEQ ID NO: 35:
   (i) SEQUENCE CHARACTERISTICS:
      (A) LENGTH: 21
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 35:
   AATCATGACT TCAAGAGTTC T 21
(2) INFORMATION FOR SEQ ID NO: 36:
   (i) SEQUENCE CHARACTERISTICS:
      (A) LENGTH: 21
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 36:
   TGAAGCAATC ATGACTTCAA G 21
(2) INFORMATION FOR SEQ ID NO: 37:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 37:
   TATAGGAGTT TTGATGTGAA 20
(2) INFORMATION FOR SEQ ID NO: 38:
   (i) SEQUENCE CHARACTERISTICS:
      (A) LENGTH: 21
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 38:
   ACAATGAGGG GGTAATCTAC A 21
(2) INFORMATION FOR SEQ ID NO: 39:
   (i) SEQUENCE CHARACTERISTICS:
      (A) LENGTH: 21
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 39:
   GACAATATAC AAACCTTCCA T 21
(2) INFORMATION FOR SEQ ID NO: 40:
   (i) SEQUENCE CHARACTERISTICS:
      (A) LENGTH: 21
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 40:
   CCAGGCATTT TAAGTTGCTG T 21
(2) INFORMATION FOR SEQ ID NO: 41:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 41:
   CCTGAAGCCA GTGAGGCCCG 20
(2) INFORMATION FOR SEQ ID NO: 42:
   (i) SEQUENCE CHARACTERISTICS:
      (A) LENGTH: 21
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 42:
   GATGAGAAAA TAGTGGAACC A 21
(2) INFORMATION FOR SEQ ID NO: 43:
   (i) SEQUENCE CHARACTERISTICS:
      (A) LENGTH: 19
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 43:
   CTGAGCAAGA TATCTAGAT 19
(2) INFORMATION FOR SEQ ID NO: 44:
   (i) SEQUENCE CHARACTERISTICS:
      (A) LENGTH: 19
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 44:
   CTACACTTTT GATTTCTGT 19
(2) INFORMATION FOR SEQ ID NO: 45:
   (i) SEQUENCE CHARACTERISTICS:
      (A) LENGTH: 22
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 45:
   TTGAACATAT CAAGCATTAG CT 22
(2) INFORMATION FOR SEQ ID NO: 46:
   (i) SEQUENCE CHARACTERISTICS:
      (A) LENGTH: 22
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 46:
   TTTACATATG TACAAATTAT GT 22
(2) INFORMATION FOR SEQ ID NO: 47:
   (i) SEQUENCE CHARACTERISTICS:
      (A) LENGTH: 22
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 47:
   AATTATCACT TTACTATACA AA 22
(2) INFORMATION FOR SEQ ID NO: 48:
   (i) SEQUENCE CHARACTERISTICS:
      (A) LENGTH: 21
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 48:
   AGGGCTGACC AAGACGGTTG T 21
(2) INFORMATION FOR SEQ ID NO: 49:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 49:
   CCATCTTCCC AGGCATTTTA 20
(2) INFORMATION FOR SEQ ID NO: 50:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 50:
   AACCCAGTGC TCCCTTTGCT 20
(2) INFORMATION FOR SEQ ID NO: 51:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 51:
   GGCCACATTG GGAAAGTTGC 20
(2) INFORMATION FOR SEQ ID NO: 52:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 52:
   GAAGTCAGCC AAGAACAGCT 20
(2) INFORMATION FOR SEQ ID NO: 53:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 53:
   ACAGGATCTC TCAGGTGGGT 20
(2) INFORMATION FOR SEQ ID NO: 54:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 54:
   CCAAAGTGAG AGCTGAGAGA 20
(2) INFORMATION FOR SEQ ID NO: 55:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 55:
   CTGATTCAAG GCTTTGGCAG 20
(2) INFORMATION FOR SEQ ID NO: 56:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 56:
   TTCCCCAGAT GCACCTGTTT 20
(2) INFORMATION FOR SEQ ID NO: 57:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 57:
   GGGCCAGAGA CCCGAGGAGA 20
(2) INFORMATION FOR SEQ ID NO: 58:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 58:
   ACGTTTGGCC TCATGGAAGT 20
(2) INFORMATION FOR SEQ ID NO: 59:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 59:
   GGAATGCAAA GCACATCCAT 20
(2) INFORMATION FOR SEQ ID NO: 60:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 60:
   CGATGCAGAT ACCGCGGAGT 20
(2) INFORMATION FOR SEQ ID NO: 61:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 61:
   GCCTGGGAGG GTATTCAGCT 20
(2) INFORMATION FOR SEQ ID NO: 62:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 62:
   CCTGTGTGTG CCTGGGAGGG 20
(2) INFORMATION FOR SEQ ID NO: 63:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 63:
   GGCATTTTAA GTTGCTGTCG 20
(2) INFORMATION FOR SEQ ID NO: 64:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 64:
   CAGCCTGCCT TACTGTGGGC 20
(2) INFORMATION FOR SEQ ID NO: 65:
   (i) SEQUENCE CHARACTERISTICS:
      (A) LENGTH: 21
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 65:
   CTTGAACAAT TAATTCCACC T 21
(2) INFORMATION FOR SEQ ID NO: 66:
   (i) SEQUENCE CHARACTERISTICS:
      (A) LENGTH: 21
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 66:
   TTACCATTGA CATAAAGTGT T 21
(2) INFORMATION FOR SEQ ID NO: 67:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 67:
   CTGTGTCTCC TGTCTCCGCT 20
(2) INFORMATION FOR SEQ ID NO: 68:
   (i) SEQUENCE CHARACTERISTICS:
      (A) LENGTH: 21
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 68:
(2) INFORMATION FOR SEQ ID NO: 69:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 69:
   TGAACATATC AAGCATTAGC 20
(2) INFORMATION FOR SEQ ID NO: 70:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 70:
   GCAATCTTGC TATGGCATAA 20
(2) INFORMATION FOR SEQ ID NO: 71:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 71:
   CCCGGCATCT TTACAAAACC 20
(2) INFORMATION FOR SEQ ID NO: 72:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 72:
   AACATCTCCG TACCATGCCA 20
(2) INFORMATION FOR SEQ ID NO: 73:
   (i) SEQUENCE CHARACTERISTICS:
      (A) LENGTH: 22
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 73:
   TCACTGCTGC CTCTGTCTCA GG 22
(2) INFORMATION FOR SEQ ID NO: 74:
   (i) SEQUENCE CHARACTERISTICS:
      (A) LENGTH: 23
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 74:
   TGATTCTTTT GAACTTAAAA GGA 23
(2) INFORMATION FOR SEQ ID NO: 75:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 75:
   TTAAAGGATG TAAGAAGGCT 20
(2) INFORMATION FOR SEQ ID NO: 76:
   (i) SEQUENCE CHARACTERISTICS:
      (A) LENGTH: 19
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 76:
   CATAAGCACA TTTATTGTC 19
(2) INFORMATION FOR SEQ ID NO: 77:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 77:
   TTTTGGGAAG CAGTTGTTCA 20
(2) INFORMATION FOR SEQ ID NO: 78:
   (i) SEQUENCE CHARACTERISTICS:
      (A) LENGTH: 21
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 78:
   AACTGTGAAG CAATCATGAC T 21
(2) INFORMATION FOR SEQ ID NO: 79:
   (i) SEQUENCE CHARACTERISTICS:
      (A) LENGTH: 22
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 79:
   CCTTGAGTGG TGCATTCAAC CT 22
(2) INFORMATION FOR SEQ ID NO: 80:

(1) SEQUENCE CHARACTERISTICS:
   (A) LENGTH: 22
   (B) TYPE: Nucleic Acid
   (C) STRANDEDNESS: Single
   (D) TOPOLOGY: Linear
      (iv) ANTI-SENSE: Yes
      (xi) SEQUENCE DESCRIPTION: SEQ ID NO: 80:
   AATGCTTGCT CACACAGGCA TT 22
(2) INFORMATION FOR SEQ ID NO: 81:
   (i) SEQUENCE CHARACTERISTICS:
      (A) LENGTH: 18
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 81:
   GCCTCGCTAT GGCTCCCA 18
(2) INFORMATION FOR SEQ ID NO: 82:
   (i) SEQUENCE CHARACTERISTICS:
      (A) LENGTH: 18
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 82:
   CATGGCGCGG GCCGCGGG 18
(2) INFORMATION FOR SEQ ID NO: 83:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 83:
   TGCATCCCCC AGGCCACCAT 20
(2) INFORMATION FOR SEQ ID NO: 84:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 84:
   TCTGAGTAGC AGAGGAGCTC 20
(2) INFORMATION FOR SEQ ID NO: 85:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 85:
   TATGTCTCCC CCACCACTTC 20

## Description

### FIELD OF THE INVENTION

This invention relates to diagnostics, research reagents and therapies for disease states which respond to modulation of the synthesis or metabolism of cell adhesion molecules. In particular, this invention relates to antisense oligonucleotide interactions with certain messenger ribonucleic acids (mRNAs) or DNAs involved in the synthesis of proteins that regulate adhesion of white blood cells to other white blood cells and to other cell types. Antisense oligonucleotides designed to hybridize to the mRNA encoding intercellular adhesion molecule-1 (ICAM-1), endothelial leukocyte adhesion molecule-1 (ELAM-1, also known as E-selectin), and vascular cell adhesion molecule-1 (VCAM-1) are provided. These oligonucleotides have been found to lead to the modulation of the activity of the RNA or DNA, and thus to the modulation of the synthesis and metabolism of specific cell adhesion molecules. Palliation and therapeutic effect result.

### BACKGROUND OF THE INVENTION

Inflammation is a localized protective response elicited by tissues in response to injury, infection, or tissue destruction resulting in the destruction of the infectious or injurious agent and isolation of the injured tissue. A typical inflammatory response proceeds as follows: recognition of an antigen as foreign or recognition of tissue damage, synthesis and release of soluble inflammatory mediators, recruitment of inflammatory cells to the site of infection or tissue damage, destruction and removal of the invading organism or damaged tissue, and deactivation of the system once the invading organism or damage has been resolved. In many human diseases with an inflammatory component, the normal, homeostatic mechanisms which attenuate the inflammatory responses are defective, resulting in damage and destruction of normal tissue.

Cell-cell interactions are involved in the activation of the immune response at each of the stages described above. One of the earliest detectable events in a normal inflammatory response is adhesion of leukocytes to the vascular endothelium, followed by migration of leukocytes out of the vasculature to the site of infection or injury. The adhesion of these leukocytes, or white blood cells, to vascular endothelium is an obligate step in the migration out of the vasculature. Harlan, J.M., *Blood* **1985,** 65, 513-525. In general, the first inflammatory cells to appear at the site of inflammation are neutrophils followed by monocytes, and lymphocytes. Cell-cell interactions are also critical for propagation of both B-lymphocytes and T-lymphocytes resulting in enhanced humoral and cellular immune responses, respectively.

The adhesion of white blood cells to vascular endothelium and other cell types is mediated by interactions between specific proteins, termed "adhesion molecules," located on the plasma membrane of both white blood cells and vascular endothelium. The interaction between adhesion molecules is similar to classical receptor ligand interactions with the exception that the ligand is fixed to the surface of a cell instead of being soluble. The identification of patients with a genetic defect in leukocyte adhesion has enabled investigators to identify a family of proteins responsible for adherence of white blood cells. Leukocyte adhesion deficiency (LAD) is a rare autosomal trait characterized by recurrent bacterial infections and impaired pus formation and wound healing. The defect was shown to occur in the common B-subunit of three heterodimeric glycoproteins, Mac-1, LFA-1, and p150,95, normally expressed on the outer cell membrane of white blood cells. Anderson and Springer, Ann. Rev. Med. **1987**, 38, 175-194. Patients suffering from LAD exhibit a defect in a wide spectrum of adherence-dependent functions of granulocytes, monocytes, and lymphocytes. Three ligands for LFA-1 have been identified, intercellular adhesion molecules 1, 2 and 3 (ICAM-1, ICAM-2 and ICAM-3). Both Mac-1 and p150,95 bind complement fragment C3bi and perhaps other unidentified ligands. Mac-1 also binds ICAM-1.

Other adhesion molecules have been identified which are involved in the adherence of white blood cells to vascular endothelium and subsequent migration out of the vasculature. These include endothelial leukocyte adhesion molecule-1 (ELAM-1), vascular cell adhesion molecule-1 (VCAM-1) and granule membrane protein-140 (GMP-140) and their respective receptors. The adherence of white blood cells to vascular endothelium appears to be mediated in part if not *in toto* by the five cell adhesion molecules ICAM-1, ICAM-2, ELAM-1, VCAM-1 and GMP-140. Dustin and Springer, *J. Cell Biol.* **1987,** 107, 321-331. Expression on the cell surface of ICAM-1, ELAM-1, VCAM-1 and GMP-140 adhesion molecules is induced by inflammatory stimuli. In contrast, expression of ICAM-2 appears to be constitutive and not sensitive to induction by cytokines. In general, GMP-140 is induced by autocoids such as histamine, leukotriene B₄, platelet activating factor, and thrombin. Maximal expression on endothelial cells is observed 30 minutes to 1 hour after stimulation, and returns to baseline within 2 to 3 hours. The expression of ELAM-1 and VCAM-1 on endothelial cells is induced by cytokines such as interleukin-1β and tumor necrosis factor, but not gamma-interferon. Maximal expression of ELAM-1 on the surface of endothelial cells occurs 4 to 6 hours after stimulation, and returns to baseline by 16 hours. ELAM-1 expression is dependent on new mRNA and protein synthesis. Elevated VCAM-1 expression is detectable 2 hours following treatment with tumor necrosis factor, is maximal 8 hours following stimulation, and remains elevated for at least 48 hours following stimulation. Rice and Bevilacqua, *Science* **1989,** 246, 1303-1306. ICAM-1 expression on endothelial cells is induced by cytokines interleukin-1 tumor necrosis factor and gamma-interferon. Maximal expression of ICAM-1 follows that of ELAM-1 occurring 8 to 10 hours after stimulation and remains elevated for at least 48 hours.

GMP-140 and ELAM-1 are primarily involved in the adhesion of neutrophils to vascular endothelial cells. VCAM-1 primarily binds T and B lymphocytes. In addition, VCAM-1 may play a role in the metastasis of melanoma, and possibly other cancers. ICAM-1 plays a role in adhesion of neutrophils to vascular endothelium, as well as adhesion of monocytes and lymphocytes to vascular endothelium, tissue fibroblasts and epidermal keratinocytes. ICAM-1 also plays a role in T-cell recognition of antigen presenting cell, lysis of target cells by natural killer cells, lymphocyte activation and proliferation, and maturation of T cells in the thymus. In addition, recent data have demonstrated that ICAM-1 is the cellular receptor for the major serotype of rhinovirus, which account for greater than 50% of common colds. Staunton et al., *Cell* **1989**, 56, 849-853; Greve et al., *Cell* **1989**, 56, 839-847.

Expression of ICAM-1 has been associated with a variety of inflammatory skin disorders such as allergic contact dermatitis, fixed drug eruption, lichen planus, and psoriasis; Ho et al., *J. Am. Acad. Dermatol*. **1990,** 22, 64-68; Griffiths and Nickoloff, *Am. J. Pathology* **1989,** 135, 1045-1053; Lisby et al., *Br. J. Dermatol.* **1989,** 120, 479-484; Shiohara et al., *Arch. Dermatol.* **1989,** 125, 1371-1376. In addition, ICAM-1 expression has been detected in the synovium of patients with rheumatoid arthritis; Hale et al., *Arth. Rheum.* **1989,** 32, 22-30, pancreatic B-cells in diabetes; Campbell et al., *Proc. Natl. Acad. Sci. U.S.A.* **1989,** 86, 4282-4286; thyroid follicular cells in patients with Graves' disease; Weetman et al., *J. Endocrinol*. **1989,** 122, 185-191; and with renal and liver allograft rejection; Faull and Russ, *Transplantation* **1989,** 48, 226-230; Adams et al., *Lancet* **1989,** 1122-1125.

It is has been hoped that inhibitors of ICAM-1, VCAM-1 and ELAM-1 expression would provide a novel therapeutic class of anti-inflammatory agents with activity towards a variety of inflammatory diseases or diseases with an inflammatory component such as asthma, rheumatoid arthritis, allograft rejections, inflammatory bowel disease, various dermatological conditions, and psoriasis. In addition, inhibitors of ICAM-1, VCAM-1, and SLAM-1 may also be effective in the treatment of colds due to rhinovirus infection, AIDS, Kaposi's sarcoma and some cancers and their metastasis. To date, there are no known therapeutic agents which effectively prevent the expression of the cellular adhesion molecules ELAM-1, VCAM-1 and ICAM-1. The use of neutralizing monoclonal antibodies against ICAM-1 in animal models provide evidence that such inhibitors if identified would have therapeutic benefit for asthma; Wegner et al., *Science* **1990**, 247, 456-459, renal allografts; Cosimi et al., *J. Immunol*. **1990**, 144, 4604-4612, and cardiac allografts; Isobe et al., *Science* **1992**, 255, 1125-1127. The use of a soluble form of ICAM-1 molecule was also effective in preventing rhinovirus infection of cells in culture. Marlin et al., *Nature* **1990**, 344, 70-72.

Current agents which affect intercellular adhesion molecules include synthetic peptides, monoclonal antibodies, and soluble forms of the adhesion molecules. To date, synthetic peptides which block the interactions with VCAM-1 or ELAM-1 have not been identified. Monoclonal antibodies may prove to be useful for the treatment of acute inflammatory response due to expression of ICAM-1, VCAM-1 and ELAM-1. However, with chronic treatment, the host animal develops antibodies against the monoclonal antibodies thereby limiting their usefulness. In addition, monoclonal antibodies are large proteins which may have difficulty in gaining access to the inflammatory site. Soluble forms of the cell adhesion molecules suffer from many of the same limitations as monoclonal antibodies in addition to the expense of their production and their low binding affinity. Thus, there is a long felt need for molecules which effectively inhibit intercellular adhesion molecules. Antisense oligonucleotides avoid many of the pitfalls of current agents used to block the effects of ICAM-1, VCAM-1 and ELAM-1.

PCT/US90/02357 (Hession et al.) discloses DNA sequences encoding Endothelial Adhesion Molecules (ELAMs), including ELAM-1 and VCAM-1 and VCAM-lb. A number of uses for these DNA sequences are provided, including (1) production of monoclonal antibody preparations that are reactive for these molecules which may be used as therapeutic agents to inhibit leukocyte binding to endothelial cells; (2) production of ELAM peptides to bind to the ELAM ligand on leukocytes which, in turn, may bind to ELAM on endothelial cells, inhibiting leukocyte binding to endothelial cells; (3) use of molecules binding to ELAMS (such as anti-ELAM antibodies, or markers such as the ligand or fragments of it) to detect inflammation; (4) use of ELAM and ELAM ligand DNA sequences to produce nucleic acid molecules that intervene in ELAM or ELAM ligand expression at the translational level using antisense nucleic acid and ribozymes to block translation of a specific MRNA either by masking MRNA with antisense nucleic acid or cleaving it with a ribozyme. It is disclosed that coding regions are the targets of choice. For VCAM-1, AUG is believed to be most likely; a 15-mer hybridizing to the AUG site is specifically disclosed in Example 17.

### OBJECTS OF THE INVENTION

It is a principle object of the invention to provide therapies for diseases with an immunological component, allografts, cancers and metastasis, inflammatory bowel disease, psoriasis and other skin diseases, colds, and AIDS through perturbation in the synthesis and expression of inflammatory cell adhesion molecules.

It is a further object of the invention to provide antisense oligonucleotides which are capable of inhibiting the function of nucleic acids encoding VCAM-1.

Yet another object is to provide means for diagnosis of dysfunctions of intercellular adhesion.

These and other objects of this invention will become apparent from a review of the instant specification.

### BRIEF DESCRIPTION OF THE DRAWINGS

FIGURE 1 is the mRNA sequence of human intercellular adhesion molecule-1 (ICAM-1).

FIGURE 2 is the mRNA sequence of human endothelial leukocyte adhesion molecule-1 (ELAM-1).

FIGURE 3 is the mRNA sequence of human vascular cell adhesion molecule-1 (VCAM-1).

FIGURE 4 is a graphical representation of the induction of ICAM-1 expression on the cell surface of various human cell lines by interleukin-1 and tumor necrosis factor.

FIGURE 5 is a graphical representation of the effects of selected antisense oligonucleotides on ICAM-1 expression on human umbilical vein endothelial cells.

FIGURE 6A and 6B are a graphical representation of the effects of an antisense oligonucleotide on the expression of ICAM-1 in human umbilical vein endothelial cells stimulated with tumor necrosis factor and interleukin-1.

FIGURE 7 is a graphical representation of the effect of antisense oligonucleotides on ICAM-1 mediated adhesion of DMSO differentiated HL-60 cells to control and tumor necrosis factor treated human umbilical vein endothelial cells.

FIGURE 8 is a graphical representation of the effects of selected antisense oligonucleotides on ICAM-1 expression in A549 human lung carcinoma cells.

FIGURE 9 is a graphical representation of the effects of selected antisense oligonucleotides on ICAM-1 expression in primary human keratinocytes.

FIGURE 10 is a graphical representation of the effects of selected antisense oligonucleotides on ELAM-1 expression on tumor necrosis factor-treated human umbilical vein endothelial cells.

### SUMMARY OF THE INVENTION

In accordance with the present invention, oligonucleotides, as defined in claim 1 hereinafter, are provided which specifically hybridize with nucleic acids encoding vascular cell adhesion molecule-1 (VCAM-1). The oligonucleotides are designed to bind either directly to mRNA or to a selected DNA portion forming a triple stranded structure, thereby modulating the amount of mRNA made from the gene.

The former relationship is commonly denoted as "antisense." The oligonucleotides are able to inhibit the function of RNA or DNA, either its translation into protein, its translocation into the cytoplasm, or any other activity necessary to its overall biological function. The failure of the RNA or DNA to perform all or part of its function results in failure of a portion of the genome controlling cell adhesion molecules to be properly expressed, thus modulating said metabolism.

It is preferred to target specific genes for antisense attack. It has been discovered that the genes coding for VCAM-1 are particularly useful for this approach. Inhibition of VCAM-1 expression is expected to be useful for the treatment of inflammatory diseases, diseases with an inflammatory component, allograft rejection, psoriasis and other skin diseases, inflammatory bowel disease, cancers and their metastasis, and viral infections.

Methods of modulating cell adhesion comprising contacting the animal with an oligonucleotide, as defined in claim 1 hereinafter, hybridizable with nucleic acids encoding VCAM-1 are provided. Methods for diagnosis are also a part of this invention.

### DETAILED DESCRIPTION OF PREFERRED EMBODIMENTS

Antisense oligonucleotides hold great promise as therapeutic agents for the treatment of many human diseases. Oligonucleotides specifically bind to the complementary sequence of either pre-mRNA or mature mRNA, as defined by Watson-Crick base pairing, inhibiting the flow of genetic information from DNA to protein. The properties of antisense oligonucleotides which make them specific for their target sequence also make them extraordinarily versatile. Because antisense oligonucleotides are long chains of four monomeric units they may be readily synthesized for any target RNA sequence. Numerous recent studies have documented the utility of antisense oligonucleotides as biochemical tools for studying target proteins. Rothenberg et al., *J. Natl. Cancer Inst.* **1989,** 81, 1539-1544; Zon, *G. Pharmaceutical Res.* **1988**, 5, 539-549). Because of recent advances in synthesis of nuclease resistant oligonucleotides, which exhibit enhanced cell uptake, it is now possible to consider the use of antisense oligonucleotides as a novel form of therapeutics.

Antisense oligonucleotides offer an ideal solution to the problems encountered in prior art approaches. They can be designed to selectively inhibit a given isoenzyme, they inhibit the production of the enzyme, and they avoid non-specific mechanisms such as free radical scavenging or binding to multiple receptors. A complete understanding of enzyme mechanisms or receptor-ligand interactions is not needed to design specific inhibitors.

### DESCRIPTION OF TARGETS

The acute infiltration of neutrophils into the site of inflammation appears to be due to increased expression of GMP-140, ELAM-1 and ICAM-1 on the surface of endothelial cells. The appearance of lymphocytes and monocytes during the later stages of an inflammatory reaction appear to be mediated by VCAM-1 and ICAM-1. ELAM-1 and GMP-140 are transiently expressed on vascular endothelial cells, while VCAM-1 and ICAM-1 are chronically expressed.

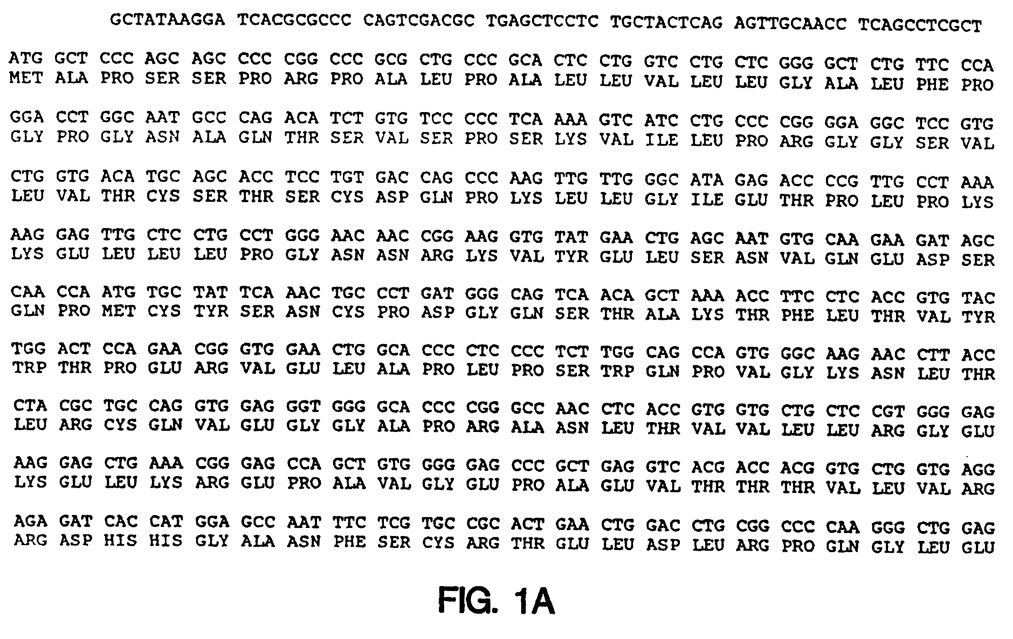

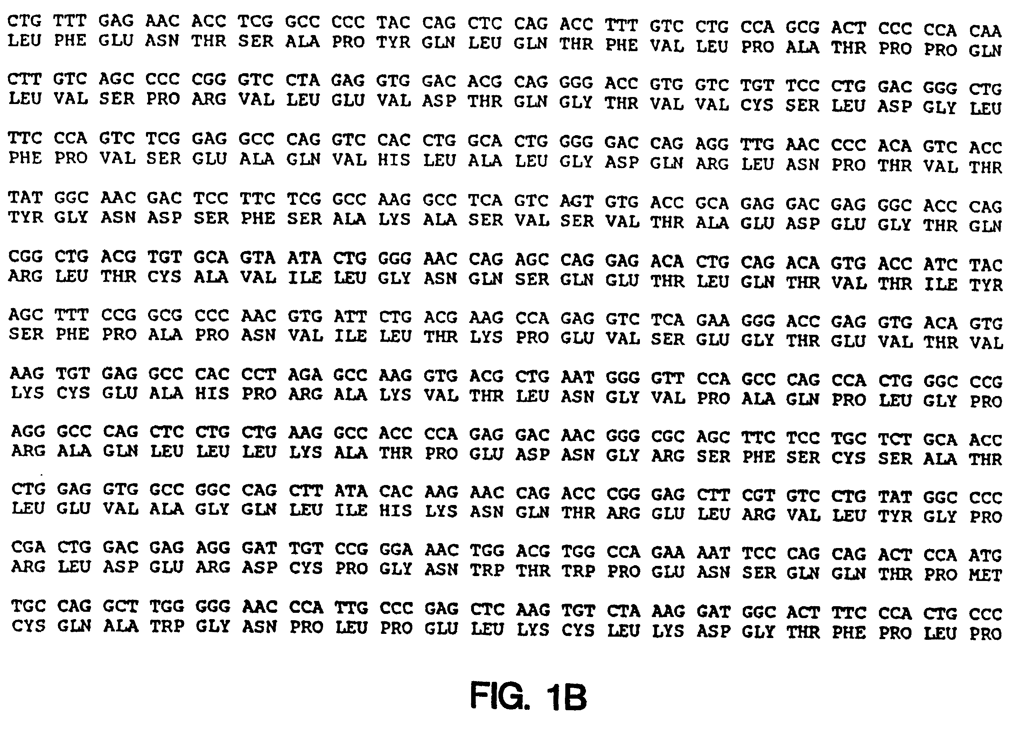

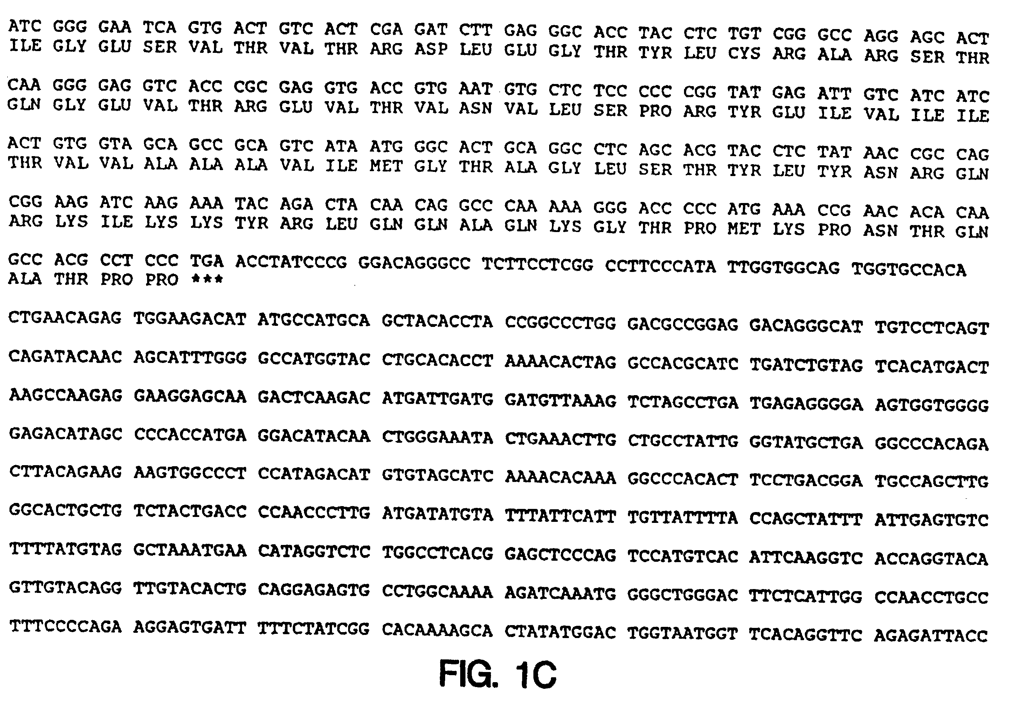

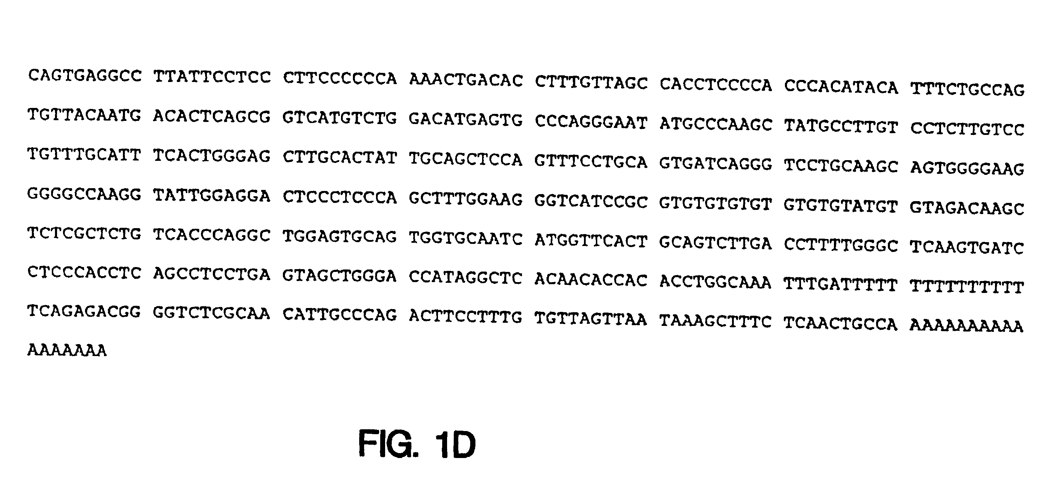

Human ICAM-1 is encoded by a 3.3-kb mRNA resulting in the synthesis of a 55,219 dalton protein (Figure 1). ICAM-1 is heavily glycosylated through N-linked glycosylation sites. The mature protein has an apparent molecular mass of 90 kDa as determined by SDS-polyacrylamide gel electrophoresis. Staunton et al., *Cell* **1988,** 52, 925-933. ICAM-1 is a member of the immunoglobulin supergene family, containing 5 immunoglobulin-like domains at the amino terminus, followed by a transmembrane domain and a cytoplasmic domain. The primary binding site for LFA-1 and rhinovirus are found in the first immunoglobulin-like domain. However, the binding sites appear to be distinct. Staunton et al., *Cell* **1990**, 61, 243-354. Recent electron micrographic studies demonstrate that ICAM-1 is a bent rod 18.7 nm in length and 2 to 3 nm in diameter. Staunton et al., *Cell* **1990**, 61, 243-254.

ICAM-1 exhibits a broad tissue and cell distribution, and may be found on white blood cells, endothelial cells, fibroblast, keratinocytes and other epithelial cells. The expression of ICAM-1 can be regulated on vascular endothelial cells, fibroblasts, keratinocytes, astrocytes and several cell lines by treatment with bacterial lipopolysaccharide and cytokines such as interleukin-1, tumor necrosis factor, gamma-interferon, and lymphotoxin. *See, e.g*., Frohman et al., *J. Neuroimmunol*. **1989,** 23, 117-124. The molecular mechanism for increased expression of ICAM-1 following cytokine treatment has not been determined.

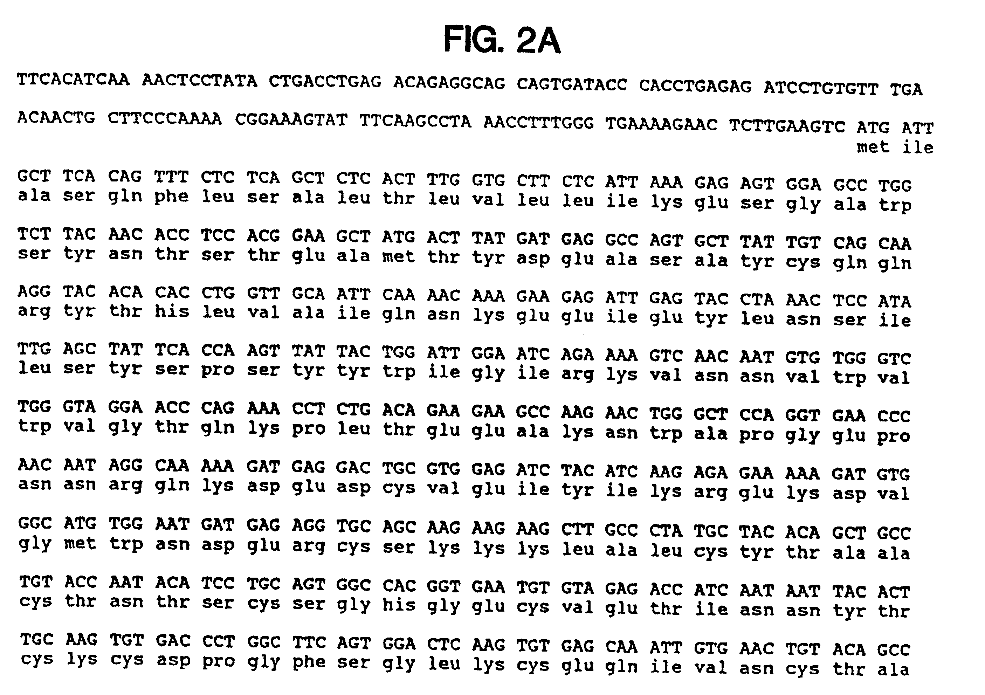

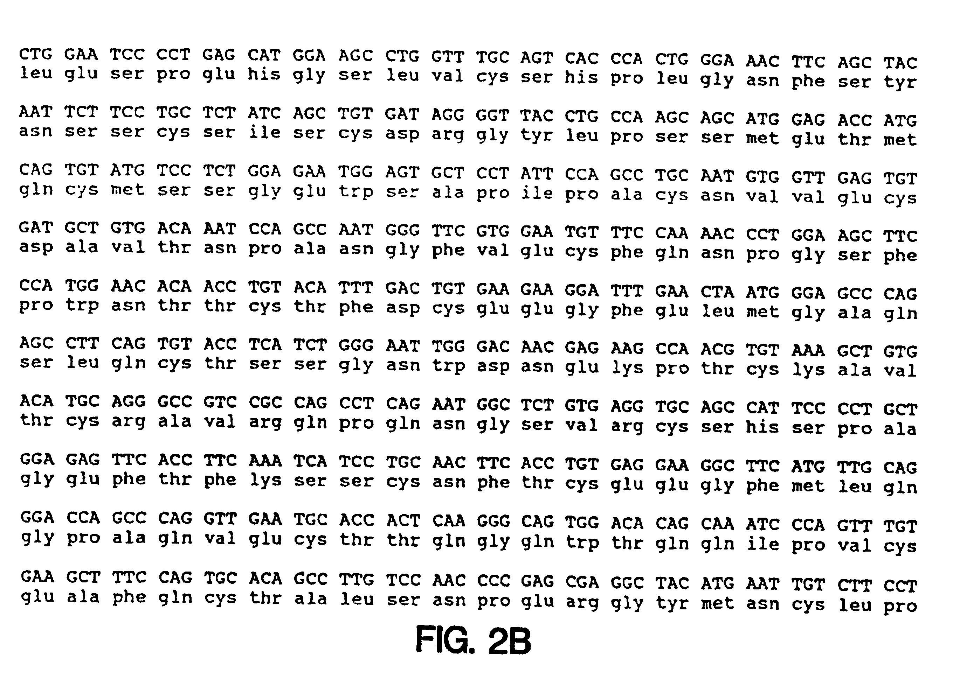

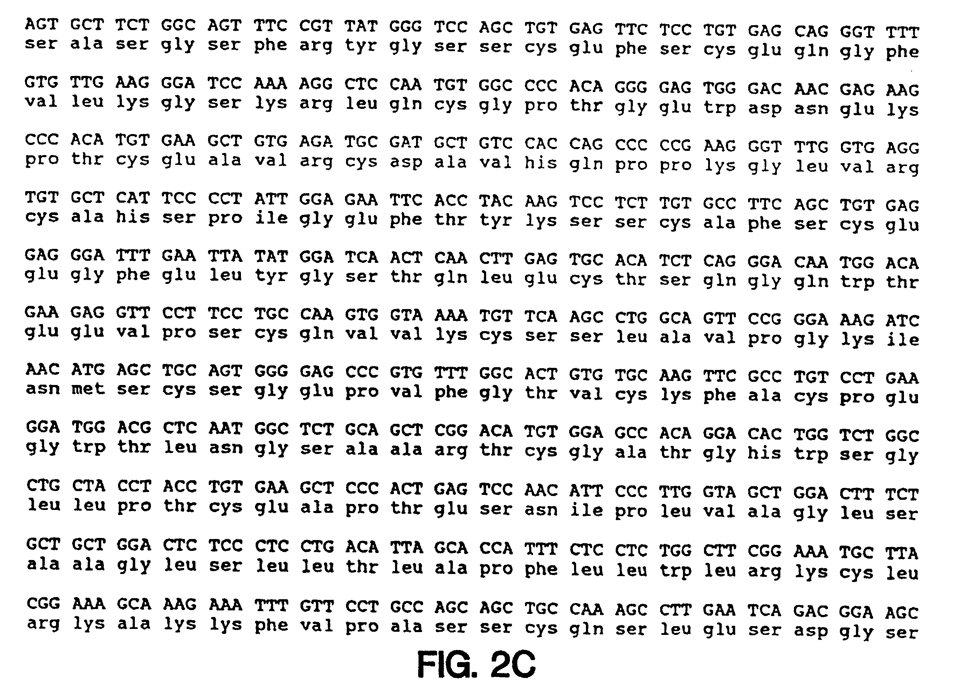

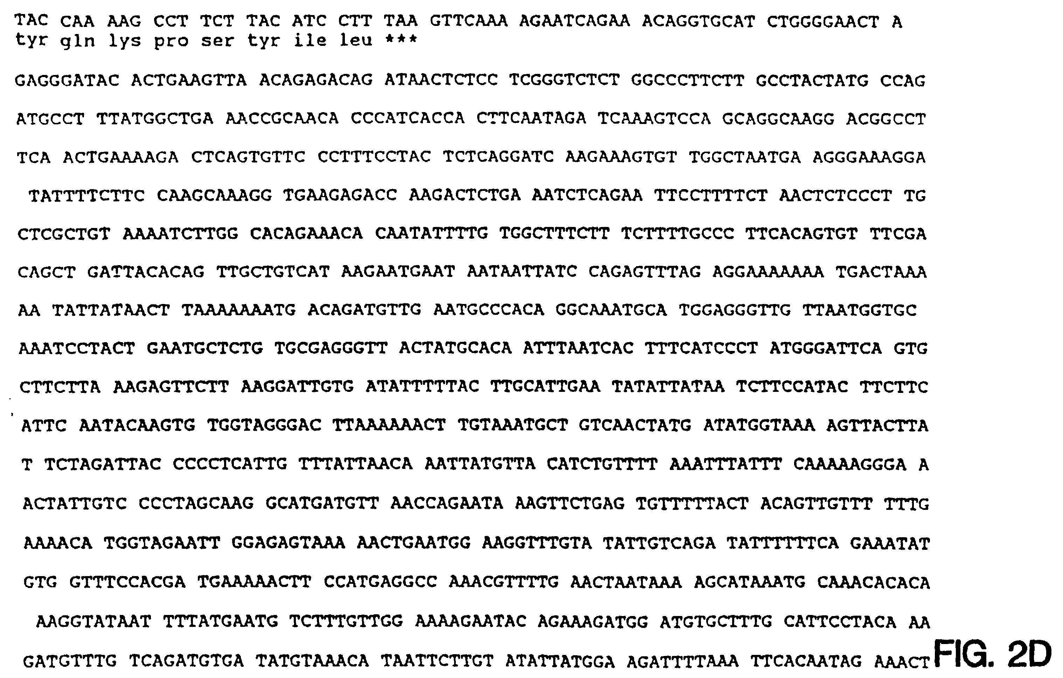

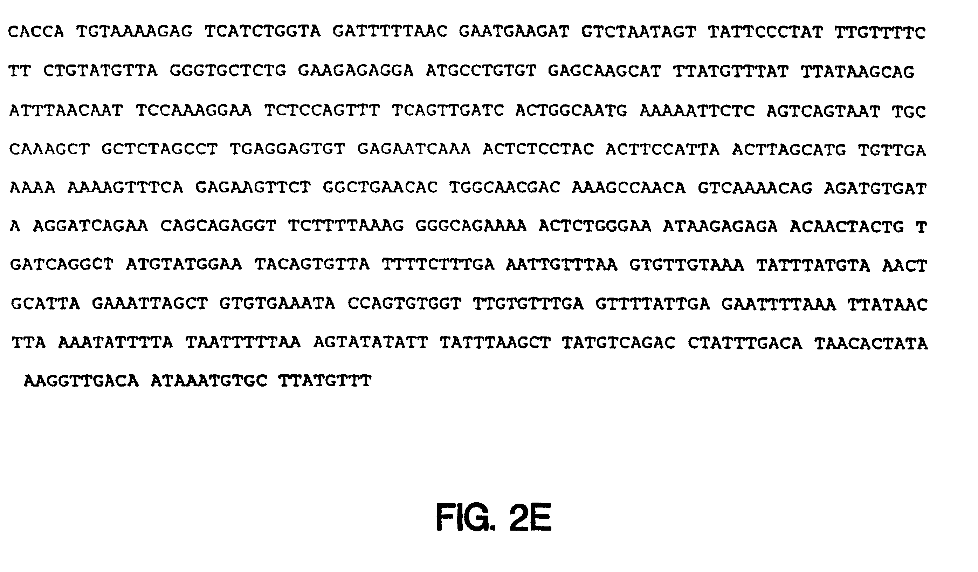

ELAM-1 is a 115-kDa membrane glycoprotein (Figure 2) which is a member of the selectin family of membrane glycoproteins. Bevilacqua et al., *Science* **1989,** 243, 1160-1165. The amino terminal region of ELAM-1 contains sequences with homologies to members of lectin-like proteins, followed by a domain similar to epidermal growth factor, followed by six tandem 60-amino acid repeats similar to those found in complement receptors 1 and 2. These features are also shared by GMP-140 and MEL-14 antigen, a lymphocyte homing antigen. ELAM-1 is encoded for by a 3.9-kb mRNA. The 3'-untranslated region of ELAM-1 mRNA contains several sequence motifs ATTTA which are responsible for the rapid turnover of cellular mRNA consistent with the transient nature of ELAM-1 expression.

ELAM-1 exhibits a limited cellular distribution in that it has only been identified on vascular endothelial cells. Like ICAM-1, ELAM-1 is inducible by a number of cytokines including tumor necrosis factor, interleukin-1 and lymphotoxin and bacterial lipopolysaccharide. In contrast to ICAM-1, ELAM-1 is not induced by gamma-interferon. Bevilacqua et al., *Proc. Natl. Acad. Sci. USA* **1987**, 84, 9238-9242; Wellicome et al., *J. Immunol.* **1990,** 144, 2558-2565. The kinetics of ELAM-1 mRNA induction and disappearance in human umbilical vein endothelial cells precedes the appearance and disappearance of ELAM-1 on the cell surface. As with ICAM-1 the molecular mechanism for ELAM-1 induction is not known.

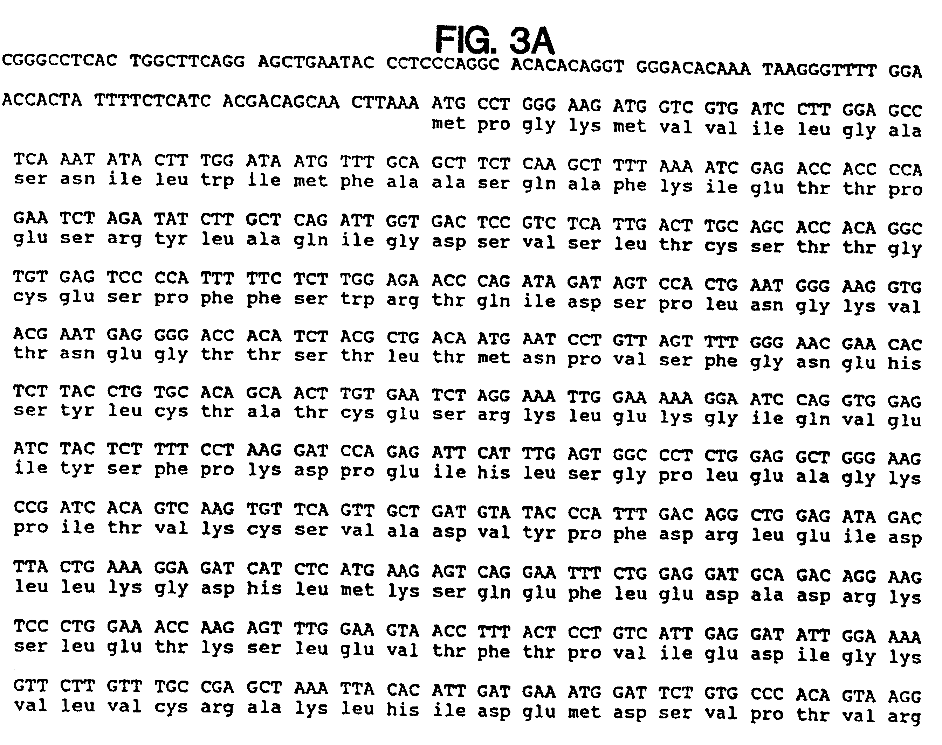

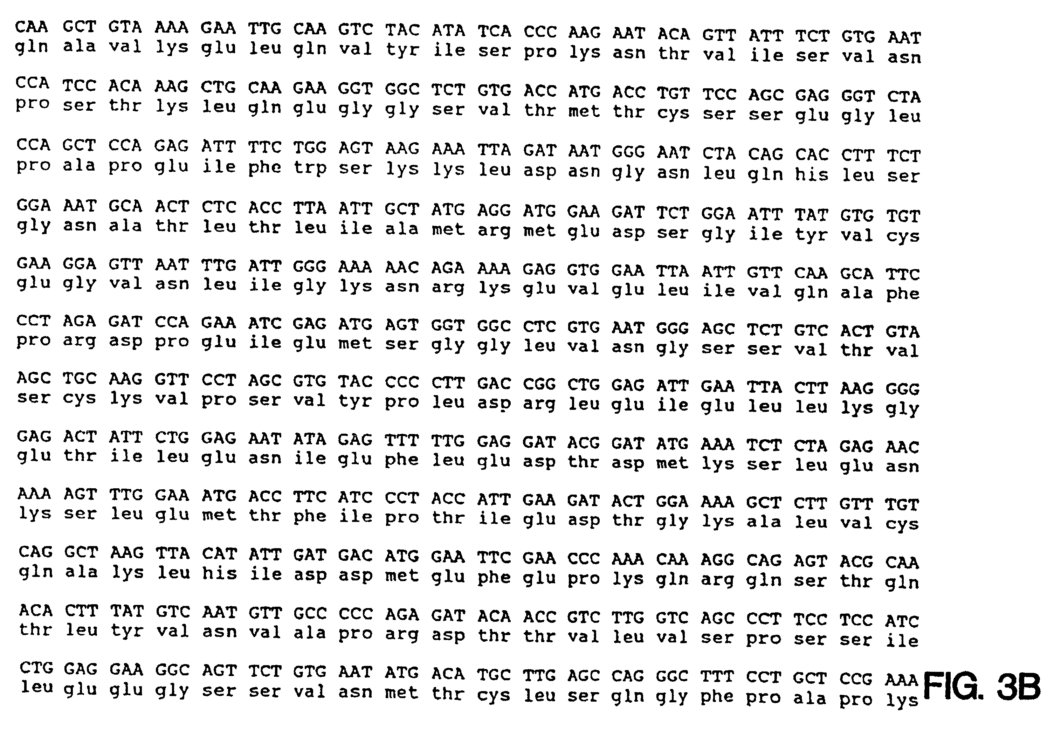

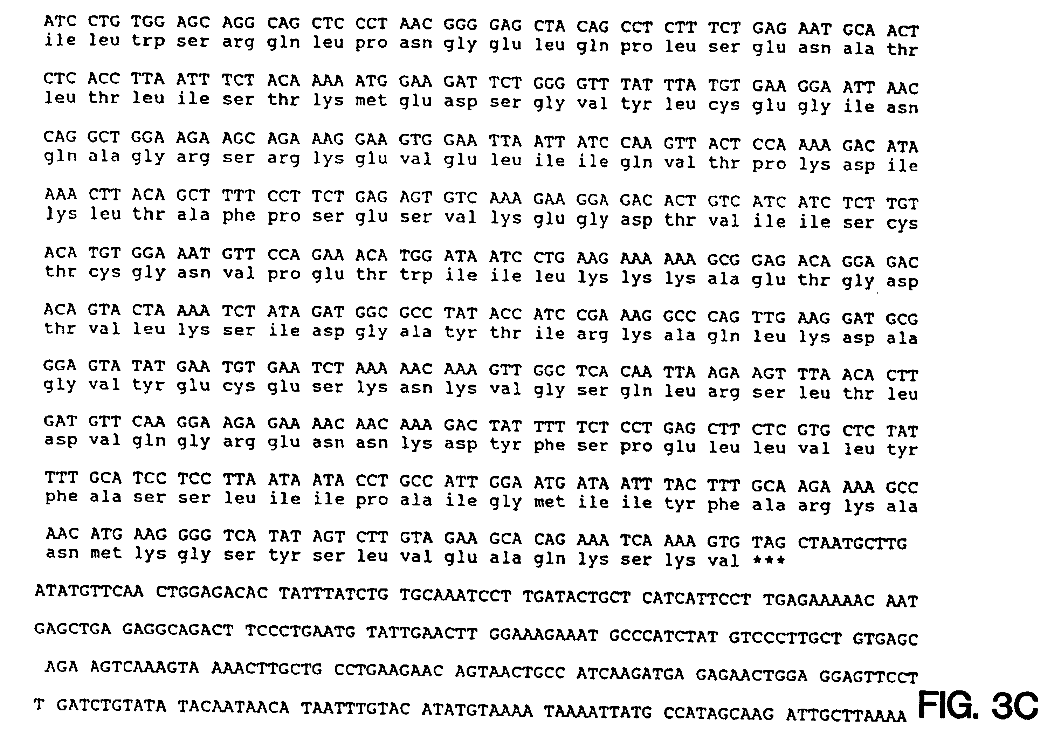

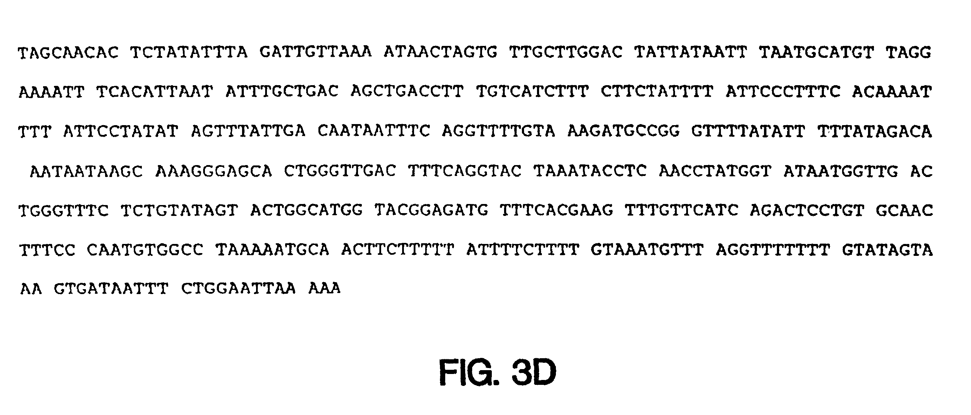

VCAM-1 is a 110-kDa membrane glycoprotein encoded by a 3.2-kb mRNA (Figure 3). VCAM-1 appears to be encoded by a single-copy gene which can undergo alternative splicing to yield products with either six or seven immunoglobulin domains. Osborn et al., *Cell* **1989,** 59, 1203-1211. The receptor for VCAM-1 is proposed to be CD29 (VLA-4) as demonstrated by the ability of monoclonal antibodies to CD29 to block adherence of Ramos cells to VCAM-1. VCAM-1 is expressed primarily on vascular endothelial cells. Like ICAM-1 and ELAM-1, expression of VCAM-1 on vascular endothelium is regulated by treatment with cytokines. Rice and Bevilacqua, *Science* **1989,** 246, 1303-1306; Rice et al., *J. Exp. Med.* **1990,** 171, 1369-1374. Increased expression appears to be due to induction of the mRNA.

For therapeutics, an animal suspected of having a disease which can be treated by decreasing the expression of ICAM-1, VCAM-1 and ELAM-1 is treated by administering oligonucleotides in accordance with this invention. Oligonucleotides may be formulated in a pharmaceutical composition, which may include carriers, thickeners, diluents, buffers, preservatives, surface active agents, liposomes or lipid formulations and the like in addition to the oligonucleotide. Pharmaceutical compositions may also include one or more active ingredients such as antimicrobial agents, anti-inflammatory agents, anesthetics, and the like in addition to oligonucleotide.

The pharmaceutical composition may be administered in a number of ways depending on whether local or systemic treatment is desired, and on the area to be treated. Administration may be topically (including ophthalmically, vaginally, rectally, intranasally), orally, by inhalation, or parenterally, for example by intravenous drip, subcutaneous, intraperitoneal or intramuscular injection.

Formulations for topical administration may include ointments, lotions, creams, gels, drops, suppositories, sprays, liquids and powders. Conventional pharmaceutical carriers, aqueous, powder or oily bases, thickeners and the like may be necessary or desirable. Coated condoms or gloves may also be useful.

Compositions for oral administration include powders or granules, suspensions or solutions in water or non-aqueous media, capsules, sachets, or tablets. Thickeners, flavorings, diluents, emulsifiers, dispersing aids or binders may be desirable.

Formulations for parenteral administration may include sterile aqueous solutions which may also contain buffers, liposomes, diluents and other suitable additives.

Dosing is dependent on severity and responsiveness of the condition to be treated, but will normally be one or more doses per day, with course of treatment lasting from several days to several months or until a cure is effected or a diminution of disease state is achieved. Persons of ordinary skill can easily determine optimum dosages, dosing methodologies and repetition rates.

The present invention employs oligonucleotides for use in antisense inhibition of the function of RNA and DNA corresponding to VCAM-1. In the context of this invention, the term "oligonucleotide" refers to an oligomer or polymer of ribonucleic acid or deoxyribonucleic acid. This term includes oligomers consisting of naturally occurring bases, sugars and intersugar (backbone) linkages as well as oligomers having non-naturally occurring portions which function similarly. Such modified or substituted oligonucleotides are often preferred over native forms because of properties such as, for example, enhanced cellular uptake and increased stability in the presence of nucleases.

Specific examples of some preferred oligonucleotides envisioned for this invention may contain phosphorothioates, phosphotriesters, methyl phosphonates, short chain alkyl or cycloalkyl intersugar linkages or short chain heteroatomic or heterocyclic intersugar linkages. Most preferred are those with CH₂-NH-O-CH₂, CH₂-N(CH₃)-O-CH₂, CH₂-O-N(CH₃)-CH₂, CH₂-N(CH₃)-N(CH₃)-CH₂ and O-N(CH₃)-CH₂-CH₂ backbones (where phosphodiester is O-P-O-CH₂). Also preferred are oligonucleotides having morpholino backbone structures. Summerton, J.E. and Weller, D.D., U.S. Patent 5,034,506. In other preferred embodiments, such as the protein-nucleic acid (PNA) backbone, the phosphodiester backbone of the oligonucleotide may be replaced with a polyamide backbone, the bases being bound directly or indirectly to the aza nitrogen atoms of the polyamide backbone. P.E. Nielsen, M. Egholm, R.H. Berg, O. Buchardt, *Science* **1991**, 254, 1497. Other preferred oligonucleotides may contain alkyl and halogen-substituted sugar moieties comprising one of the following at the 2' position: OH, SH, SCH₃, F, OCN, O(CH₂)ₙNH₂ or O(CH₂)ₙCH₃ where n is from 1 to about 10; C₁ to C₁₀ lower alkyl, substituted lower alkyl, alkaryl or aralkyl; Cl; Br; CN; CF₃; OCF₃; O-, S-, or N-alkyl; O-, S-, or N-alkenyl; SOCH₃; SO₂CH₃; ONO₂; NO₂; N₃; NH₂; heterocycloalkyl; heterocycloalkaryl; aminoalkylamino; polyalkylamino; substituted silyl; an RNA cleaving group; a conjugate; a reporter group; an intercalator; a group for improving the pharmacokinetic properties of an oligonucleotide; or a group for improving the pharmacodynamic properties of an oligonucleotide and other substituents having similar properties. Oligonucleotides may also have sugar mimetics such as cyclobutyls in place of the pentofuranosyl group.

The oligonucleotides in accordance with this invention preferably comprise from about 3 to about 50 nucleic acid base units. It is more preferred that such oligonucleotides comprise from about 8 to 25 nucleic acid base units, and still more preferred to have from about 12 to 22 nucleic acid base units. As will be appreciated, a nucleic acid base unit is a base-sugar combination suitably bound to an adjacent nucleic acid base unit through phosphodiester or other bonds.

The oligonucleotides used in accordance with this invention may be conveniently and routinely made through the well-known technique of solid phase synthesis. Equipment for such synthesis is sold by several vendors including Applied Biosystems. Any other means for such synthesis may also be employed, however the actual synthesis of the oligonucleotides are well within the talents of the routineer. It is also well known to use similar techniques to prepare other oligonucleotides such as the phosphorothioates and alkylated derivatives.

In accordance with this invention, persons of ordinary skill in the art will understand that messenger RNA identified by the open reading frames (ORFs) of the DNA from which they are transcribed includes not only the information from the ORFs of the DNA, but also associated ribonucleotides which form regions known to such persons as the 5'-untranslated region, the 3'- untranslated region, and intervening sequence ribonucleotides. Thus, oligonucleotides may be formulated in accordance with this invention which are targeted wholly or in part to these associated ribonucleotides as well as to the informational ribonucleotides. In preferred embodiments, the oligonucleotide is specifically hybridizable with a transcription initiation site, a translation initiation site, an intervening sequence and sequences in the 3'-untranslated region.

In accordance with this invention, the oligonucleotide is specifically hybridizable with portions of nucleic acids encoding a protein involved in the adhesion of white blood cells either to other white blood cells or other cell types. Said protein is vascular cell adhesion molecule-1. Oligonucleotides comprising the corresponding sequence, or part thereof, are useful in the invention.

Figure 3 is a human vascular cell adhesion molecule-1 mRNA sequence. A preferred sequence segment which may be useful in whole or in part is:

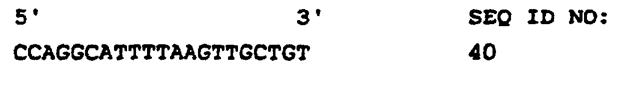

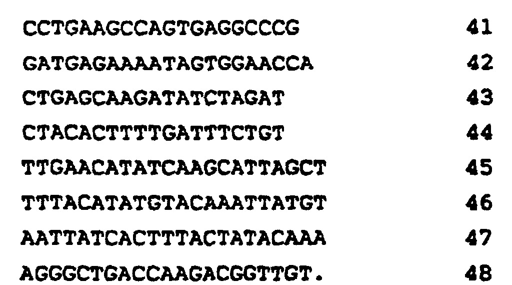

While the illustrated sequences are believed to be accurate, the present invention is directed to the correct sequences should errors be found. Oligonucleotides useful in the invention comprise one of these sequences, or part thereof. Thus, it is preferred to employ any of these oligonucleotides as set forth above or any of the similar oligonucleotides which persons of ordinary skill in the art can prepare from knowledge of the preferred antisense targets for the modulation of the synthesis of inflammatory cell adhesion molecules.

Several preferred embodiments of this invention are exemplified in accordance with the following nonlimiting examples. The target mRNA species for modulation relate to vascular cell adhesion molecule-1. Persons of ordinary skill in the art will appreciate that the present invention is not so limited, however, and that it is generally applicable. The inhibition or modulation of production of the VCAM-1 is expected to have significant therapeutic benefits in the treatment of disease. In order to assess the effectiveness of the compositions, an assay or series of assays is required.

### EXAMPLES

### Example 1

Expression of ICAM-1, VCAM-1 and ELAM-1 on the surface of cells can be quantitated using specific monoclonal antibodies in an ELISA. Cells are grown to confluence in 96 well microtiter plates. The cells are stimulated with either interleukin-1 or tumor necrosis factor for 4 to 8 hours to quantitate ELAM-1 and 8 to 24 hours to quantitate ICAM-1 and VCAM-1. Following the appropriate incubation time with the cytokine, the cells are gently washed three times with a buffered isotonic solution containing calcium and magnesium such as Dulbecco's phosphate buffered saline (D-PBS). The cells are then directly fixed on the microtiter plate with 1 to 2% paraformaldehyde diluted in D-PBS for 20 minutes at 25°C. The cells are washed again with D-PBS three times. Nonspecific binding sites on the microtiter plate are blocked with 2% bovine serum albumin in D-PBS for 1 hour at 37°C. Cells are incubated with the appropriate monoclonal antibody diluted in blocking solution for 1 hour at 37°C. Unbound antibody is removed by washing the cells three times with D-PBS. Antibody bound to the cells is detected by incubation with a 1:1000 dilution of biotinylated goat anti-mouse IgG (Bethesda Research Laboratories, Gaithersberg, MD) in blocking solution for 1 hour at 37°C. Cells are washed three times with D-PBS and then incubated with a 1:1000 dilution of streptavidin conjugated to β-galactosidase (Bethesda Research Laboratories) for 1 hour at 37°C. The cells are washed three times with D-PBS for 5 minutes each. The amount of β-galactosidase bound to the specific monoclonal antibody is determined by developing the plate in a solution of 3.3 mM chlorophenolred-β-D-galactopyranoside, 50 mM sodium phosphate, 1.5 mM MgCl₂; pH=7.2 for 2 to 15 minutes at 37°C. The concentration of the product is determined by measuring the absorbance at 575 nm in an ELISA microtiter plate reader.

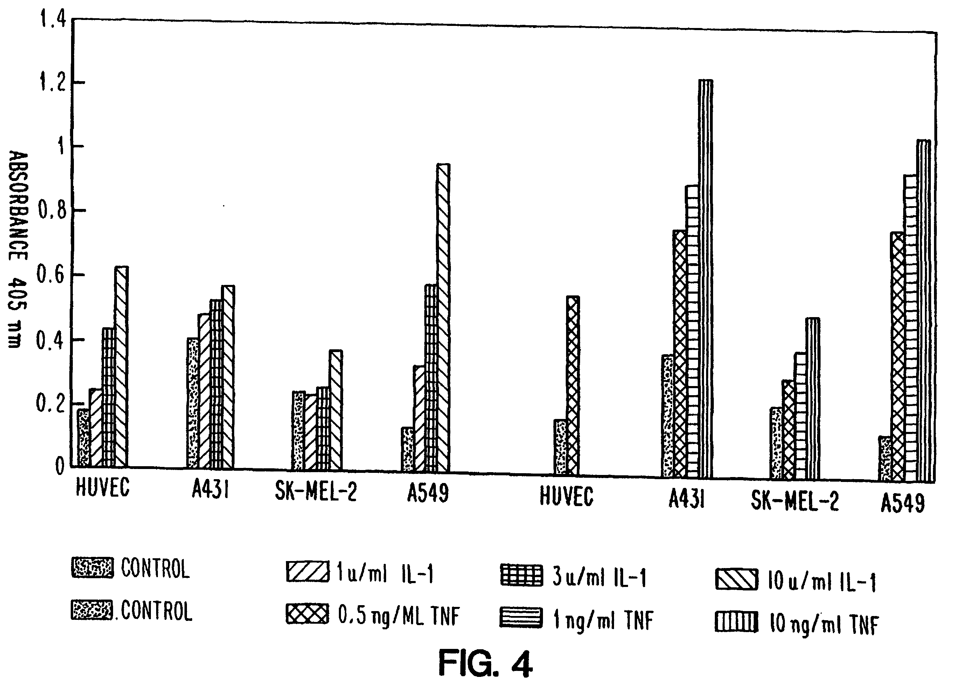

An example of the induction of ICAM-1 observed following stimulation with either interleukin-1β or tumor necrosis factor α in several human cell lines is shown in Figure 4. Cells were stimulated with increasing concentrations of interleukin-1 or tumor necrosis factor for 15 hours and processed as described above. ICAM-1 expression was determined by incubation with a 1:1000 dilution of the monoclonal antibody 84H10 (Amac Inc., Westbrook, ME). The cell lines used were passage 4 human umbilical vein endothelial cells (HUVEC), a human epidermal carcinoma cell line (A431), a human melanoma cell line (SK-MEL-2) and a human lung carcinoma cell line (A549). ICAM-1 was induced on all the cell lines, however, tumor necrosis factor was more effective than interleukin-1 in induction of ICAM-1 expression on the cell surface (Figure 4).

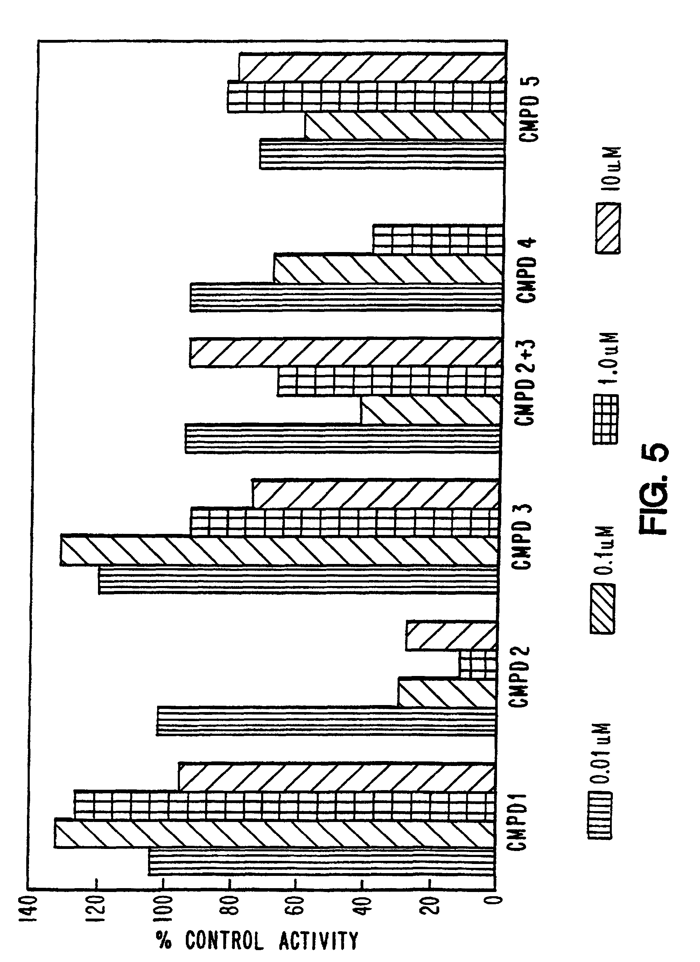

Screening antisense oligonucleotides for inhibition of ICAM-1, VCAM-1 or ELAM-1 expression is performed as described above with the exception of pretreatment of cells with the oligonucleotides prior to challenge with the cytokines. An example of antisense oligonucleotide inhibition of ICAM-1 expression is shown in Figure 5. Human umbilical vein endothelial cells (HUVEC) were treated with increasing concentration of oligonucleotide diluted in Opti MEM (GIBCO, Grand Island, NY) containing 8 µM N-[1-(2,3-dioleyloxy) propyl]-N,N,N-trimethylammonium chloride (DOTMA) for 4 hours at 37°C to enhance uptake of the oligonucleotides. The medium was removed and replaced with endothelial growth medium (EGM-UV; Clonetics, San Diego, CA) containing the indicated concentration of oligonucleotide for an additional 4 hours. Interleukin-1β was added to the cells at a concentration of 5 units/ml and incubated for 14 hours at 37°C. The cells were quantitated for ICAM-1 expression using a 1:1000 dilution of the monoclonal antibody 84H10 as described above. The oligonucleotides used were:
**COMPOUND 1** - (ISIS 1558) a phosphodiester oligonucleotide designed to hybridize with position 64-80 of the mRNA covering the AUG initiation of translation codon having the sequence
**COMPOUND 2 -** (ISIS 1570) a phosphorothioate containing oligonucleotide corresponding to the same sequence as COMPOUND 1.
**COMPOUND 3** - a phosphorothioate oligonucleotide complementary to COMPOUND 1 and COMPOUND 2 exhibiting the sequence
**COMPOUND 4** - (ISIS 1572) a phosphorothioate containing oligonucleotide designed to hybridize to positions 2190-2210 of the mRNA in the 3' untranslated region containing the sequence
**COMPOUND 5** - (ISIS 1821) a phosphorothioate containing oligonucleotide designed to hybridize to human 5-lipoxygenase mRNA used as a control containing the sequence

The phosphodiester oligonucleotide targeting the AUG initiation of translation region of the human ICAM-1 mRNA (COMPOUND 1) did not inhibit expression of ICAM-1, however, the corresponding phosphorothioate containing oligonucleotide (COMPOUND 2) inhibited ICAM-1 expression by 70% at a concentration of 0.1 µM and 90% at 1 µM concentration (Figure 4). The increased potency of the phosphorothioate oligonucleotide over the phosphodiester is probably due to increased stability. The sense strand to COMPOUND 2, COMPOUND 3, modestly inhibited ICAM-1 expression at 10 µM. If COMPOUND 2 was prehybridized to COMPOUND 3 prior to addition to the cells, the effects of COMPOUND 2 on ICAM-1 expression were attenuated suggesting that the activity of COMPOUND 2 was due to antisense oligonucleotide effect, requiring hybridization to the mRNA. The antisense oligonucleotide directed against 3' untranslated sequences (COMPOUND 4) inhibited ICAM-1 expression 62% at a concentration of 1 µM (Figure 5). The control oligonucleotide, targeting human 5-lipoxygenase (COMPOUND 5) reduced ICAM-1 expression by 20%. These data demonstrate that oligonucleotides are capable of inhibiting ICAM-1 expression on human umbilical vein endothelial cells and suggest that the inhibition of ICAM-1 expression is due to an antisense activity.

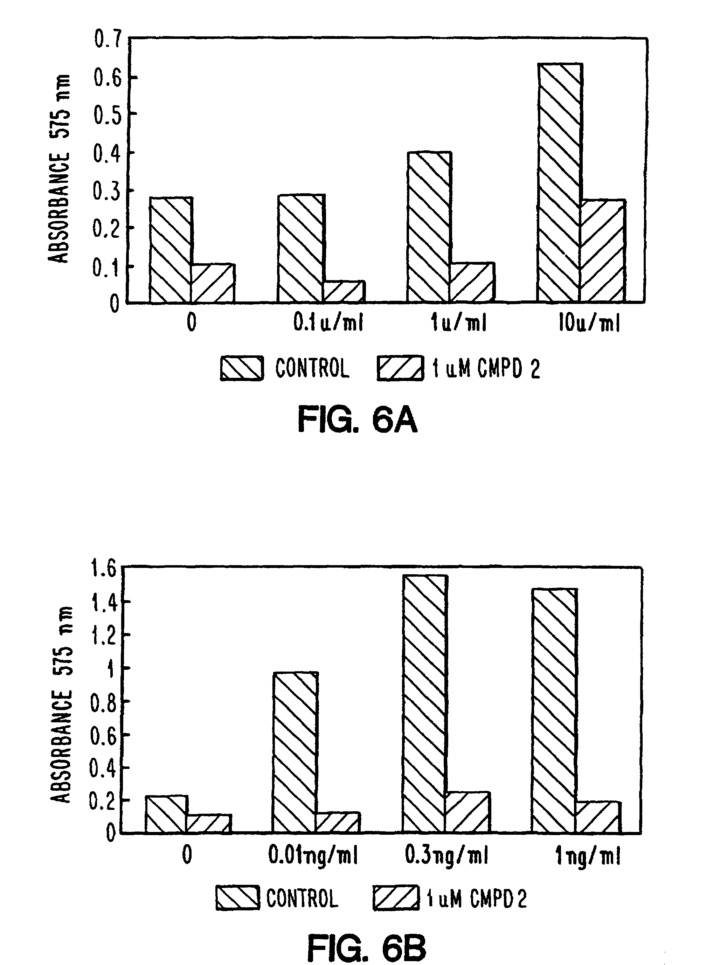

The antisense oligonucleotide COMPOUND 2 at a concentration of 1 µM inhibits expression of ICAM-1 on human umbilical vein endothelial cells stimulated with increasing concentrations of tumor necrosis factor and interleukin-1 (Figure 6). These data demonstrate that the effects of COMPOUND 2 are not specific for interleukin-1 stimulation of cells.

Analogous assays can also be used to demonstrate inhibition of ELAM-1 and VCAM-1 expression by antisense oligonucleotides.

### Example 2

A second cellular assay which can be used to demonstrate the effects of antisense oligonucleotides on ICAM-1, VCAM-1 or ELAM-1 expression is a cell adherence assay. Target cells are grown as a monolayer in a multiwell plate, treated with oligonucleotide followed by cytokine. The adhering cells are then added to the monolayer cells and incubated for 30 to 60 minutes at 37°C and washed to remove nonadhering cells. Cells adhering to the monolayer may be determined either by directly counting the adhering cells or prelabeling the cells with a radioisotope such as ⁵¹Cr and quantitating the radioactivity associated with the monolayer as described. Dustin and Springer, *J. Cell Biol.* **1988**, 107, 321-331. Antisense oligonucleotides may target either ICAM-1, VCAM-1 or ELAM-1 in the assay.

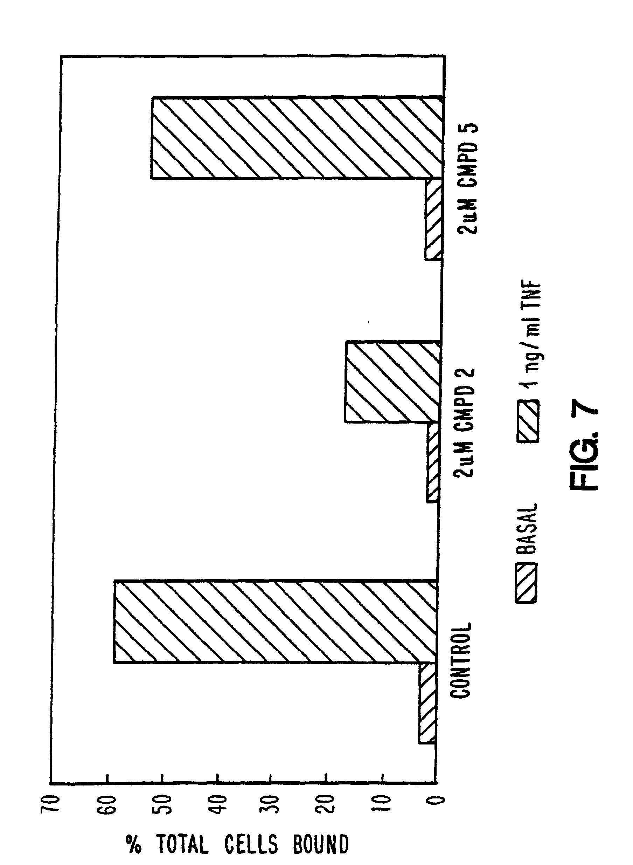

An example of the effects of antisense oligonucleotides targeting ICAM-1 mRNA on the adherence of DMSO differentiated HL-60 cells to tumor necrosis factor treated human umbilical vein endothelial cells is shown in Figure 7. Human umbilical vein endothelial cells were grown to 80% confluence in 12 well plates. The cells were treated with 2 µM oligonucleotide diluted in Opti-MEM containing 8 µM DOTMA for 4 hours at 37°C. The medium was removed and replaced with fresh endothelial cell growth medium (EGM-UV) containing 2 µM of the indicated oligonucleotide and incubated 4 hours at 37°C. Tumor necrosis factor, 1 ng/ml, was added to cells as indicated and cells incubated for an additional 19 hours. The cells were washed once with EGM-UV and 1.6 x 10⁶ HL-60 cells differentiated for 4 days with 1.3% DMSO added. The cells were allowed to attach for 1 hour at 37°C and gently washed 4 times with Dulbecco's phosphate-buffered saline (D-PBS) warmed to 37°C. Adherent cells were detached from the monolayer by addition of 0.25 ml of cold (4°C) phosphate-buffered saline containing 5 mM EDTA and incubated on ice for 5 minutes. The number of cells removed by treatment with EDTA was determined by counting with a hemocytometer. Endothelial cells detached from the monolayer by EDTA treatment could easily be distinguished from HL-60 cells by morphological differences.

In the absence of tumor necrosis factor, 3% of the HL-60 cells bound to the endothelial cells. Treatment of the endothelial cell monolayer with 1 ng/ml tumor necrosis factor increased the number of adhering cells to 59% of total cells added (Figure 7). Treatment with the antisense oligonucleotide COMPOUND 2 or the control oligonucleotide COMPOUND 5 did not change the number of cells adhering to the monolayer in the absence of tumor necrosis factor treatment (Figure 7). The antisense oligonucleotide, COMPOUND 2 reduced the number of adhering cells from 59% of total cells added to 17% of the total cells added (Figure 7). In contrast, the control oligonucleotide COMPOUND 5 did not significantly reduce the number of cells adhering to the tumor necrosis factor treated endothelial monolayer, i.e., 53% of total cells added for COMPOUND 5 treated cells versus 59% for control cells.

These data indicate that antisense oligonucleotides are capable of inhibiting ICAM-1 expression on endothelial cells and that inhibition of ICAM-1 expression correlates with a decrease in the adherence of a neutrophil-like cell to the endothelial monolayer in a sequence specific fashion. Because other molecules also mediate adherence of white blood cells to endothelial cells, such as ELAM-1, and VCAM-1 it is not expected that adherence would be completely blocked.

### Example 3

### Synthesis and characterization of oligonucleotides

Unmodified DNA oligonucleotides were synthesized on an automated DNA synthesizer (Applied Biosystems model 380B) using standard phosphoramidite chemistry with oxidation by iodine. β-cyanoethyldiisopropyl-phosphoramidites were purchased from Applied Biosystems (Foster City, CA). For phosphorothioate oligonucleotides, the standard oxidation bottle was replaced by a 0.2 M solution of 3H-1,2-benzodithiole-3-one 1,1-dioxide in acetonitrile for the stepwise thiation of the phosphite linkages. The thiation cycle wait step was increased to 68 seconds and was followed by the capping step.

2'-0-methyl phosphorothioate oligonucleotides were synthesized using 2'-O-methyl β-cyanoethyldiisopropylphosphoramidites (Chemgenes, Needham MA) and the standard cycle for unmodified oligonucleotides, except the wait step after pulse delivery of tetrazole and base was increased to 360 seconds. The 3'-base used to start the synthesis was a 2'-deoxyribonucleotide.

2'-fluoro phosphorothioate oligonucleotides were synthesized using 5'-dimethoxytrityl-3'-phosphoramidites and prepared as disclosed in U.S. patent application Serial No. 463,358, filed January 11, 1990, and 566,977, filed August 13, 1990, which are assigned to the same assignee as the instant application and which are incorporated by reference herein. The 2'-fluoro oligonucleotides were prepared using phosphoramidite chemistry and a slight modification of the standard DNA synthesis protocol: deprotection was effected using methanolic ammonia at room temperature.

After cleavage from the controlled pore glass column (Applied Biosystems) and deblocking in concentrated ammonium hydroxide at 55°C for 18 hours, the oligonucleotides were purified by precipitation twice out of 0.5 M NaCl with 2.5 volumes ethanol. Analytical gel electrophoresis was accomplished in 20% acrylamide, 8 M urea, 45 mM Tris-borate buffer, pH 7.O. Oligodeoxynucleotides and phosphorothioate oligonucleotides were judged from electrophoresis to be greater than 80% full length material.

RNA oligonucleotide synthesis was performed on an ABI model 380B DNA synthesizer. The standard synthesis cycle was modified by increasing the wait step after the pulse delivery of tetrazole to 900 seconds. The bases were deprotected by incubation in methanolic ammonia overnight. Following base deprotections the oligonucleotides were dried *in vacuo*. The t-butyldimethylsilyl protecting the 2' hydroxyl was removed by incubating the oligonucleotide in 1 M tetrabutylammonium-fluoride in tetrahydrofuran overnight. The RNA oligonucleotides were further purified on C₁₈ Sep-Pak cartridges (Waters, Division of Millipore Corp., Milford MA) and ethanol precipitated.

The relative amounts of phosphorothioate and phosphodiester linkages obtained by this synthesis were periodically checked by ³¹P NMR spectroscopy. The spectra were obtained at ambient temperature using deuterium oxide or dimethyl sulfoxide-d₆ as solvent. Phosphorothioate samples typically contained less than one percent of phosphodiester linkages.

Secondary evaluation was performed with oligonucleotides purified by trityl-on HPLC on a PRP-1 column (Hamilton Co., Reno, Nevada) using a gradient of acetonitrile in 50 mM triethylammonium acetate, pH 7.0 (4% to 32% in 30 minutes, flow rate = 1.5 ml/min). Appropriate fractions were pooled, evaporated and treated with 5% acetic acid at ambient temperature for 15 minutes. The solution was extracted with an equal volume of ethyl acetate, neutralized with ammonium hydroxide, frozen and lyophilized. HPLC-purified oligonucleotides were not significantly different in potency from precipitated oligonucleotides, as judged by the ELISA assay for ICAM-1 expression.

### Example 4

### Cell culture and treatment with oligonucleotides

The human lung carcinoma cell line A549 was obtained from the American Type Culture Collection (Bethesda MD). Cells were grown in Dulbecco's Modified Eagle's Medium (Irvine Scientific, Irvine CA) containing 1 gm glucose/liter and 10% fetal calf serum (Irvine Scientific). Human umbilical vein endothelial cells (HUVEC) (Clonetics, San Diego CA) were cultured in EGM-UV medium (Clonetics). HUVEC were used between the second and sixth passages. Human epidermal carcinoma A431 cells were obtained from the American Type Culture Collection and cultured in DMEM with 4.5 g/l glucose. Primary human keratinocytes were obtained from Clonetics and grown in KGM (Keratinocyte growth medium, Clonetics).

Cells grown in 96-well plates were washed three times with Opti-MEM (GIBCO, Grand Island, NY) prewarmed to 37°C. 100 µl of Opti-MEM containing either 10 µg/ml N-[1-(2,3-dioleyloxy)propyl]-N,N,N-trimethylammonium chloride (DOTMA, Bethesda Research Labs, Bethesda MD) in the case of HUVEC cells or 20 µg/ml DOTMA in the case of A549 cells was added to each well. Oligonucleotides were sterilized by centrifugation through 0.2 µm Centrex cellulose acetate filters (Schleicher and Schuell, Keene, NH). Oligonucleotides were added as 20x stock solution to the wells and incubated for 4 hours at 37°C. Medium was removed and replaced with 150 µl of the appropriate growth medium containing the indicated concentration of oligonucleotide. Cells were incubated for an additional 3 to 4 hours at 37°C then stimulated with the appropriate cytokine for 14 to 16 hours, as indicated. ICAM-1 expression was determined as described in Example 1. The presence of DOTMA during the first 4 hours incubation with oligonucleotide increased the potency of the oligonucleotides at least 100-fold. This increase in potency correlated with an increase in cell uptake of the oligonucleotide.

### Example 5

### ELISA screening of additional antisense oligonucleotides for activity against ICAM-1 gene expression in Interxeukin-1β-stimulated cells

Antisense oligonucleotides were originally designed that would hybridize to five target sites on the human ICAM-1 mRNA. Oligonucleotides were synthesized in both phosphodiester (P=O; ISIS 1558, 1559, 1563, 1564 and 1565) and phosphorothioate (P=S; ISIS 1570, 1571, 1572, 1573, and 1574) forms. The oligonucleotides are shown in Table 1.

**TABLE 1**

| **ANTISENSE OLIGONUCLEOTIDES WHICH TARGET HUMAN ICAM-1** | | | |
|---|---|---|---|
| **ISIS NO.** | **SEQ ID NO.** | **TARGET REGION** | **MODIFICATION** |
| 1558 | 1 | AUG Codon (64-81) | P=O |
| 1559 | 2 | 5'-Untranslated (32-49) | P=O |
| 1563 | 3 | 3'-Untranslated (2190-3010) | P=O |
| 1564 | 4 | 3'-Untranslated (2849-2866) | P=O |
| 1565 | 5 | Coding Region (1378-1395) | P=O |
| 1570 | 1 | AUG Codon (64-81) | P=S |
| 1571 | 2 | 5'-Untranslated (32-49) | P=S |
| 1572 | 3 | 3'-Untranslated (2190-3010) | P=S |
| 1573 | 4 | 3'-Untranslated (2849-2866) | P=S |
| 1574 | 5 | Coding Region (1378-1395) | P=S |
| 1930 | 6 | 5'-Untranslated (1-20) | P=S |
| 1931 | 7 | AUG Codon (55-74) | P=S |
| 1932 | 8 | AUG Codon (72-91) | P=S |
| 1933 | 9 | Coding Region (111-130) | P=S |
| 1934 | 10 | Coding Region (351-370) | P=S |
| 1935 | 11 | Coding Region (889-908) | P=S |
| 1936 | 12 | Coding Region (1459-1468) | P=S |
| 1937 | 13 | Termination Codon (1651-1687) | P=S |
| 1938 | 14 | Termination Codon (1668-1687) | P=S |
| 1939 | 15 | 3'-Untranslated (1952-1971) | P=S |
| 1940 | 16 | 3'-Untranslated (2975-2994) | P=S |
| 2149 | 17 | AUG Codon (64-77) | P=S |
| 2163 | 18 | AUG Codon (64-75) | P=S |
| 2164 | 19 | AUG Codon (64-73) | P=S |
| 2165 | 20 | AUG Codon (66-80) | P=S |
| 2173 | 21 | AUG Codon (64-79) | P=S |
| 2302 | 22 | 3'-Untranslated (2114-2133) | P=S |
| 2303 | 23 | 3'-Untranslated (2039-2058) | P=S |
| 2304 | 24 | 3'-Untranslated (1895-1914) | P=S |
| 2305 | 25 | 3'-Untranslated (1935-1954) | P=S |
| 2307 | 26 | 3'-Untranslated (1976-1995) | P=S |
| 2634 | 1 | AUG-Codon (64-81) | 2'-fluoro A,C & U; P=S |
| 2637 | 15 | 3'-Untranslated (1952-1971) | 2'-fluoro A, C & U; |
| 2691 | 1 | AUG Codon (64-81) | P=O, except last 3 bases, P=S |
| 2710 | 15 | 3'-Untranslated (1952-1971) | 2 ' - O - methyl; P=O |
| 2711 | 1 | AUG Codon (64-81) | 2 ' - O - methyl; P=O |
| 2973 | 15 | 3'-Untranslated (1952-1971) | 2 ' - O - methyl; P=S |
| 2974 | 1 | AUG Codon (64-81) | 2 ' - O - methyl; P=S |
| 3064 | 27 | 5'-CAP (32-51) | P=S; G & C added as spacer to 3' |
| 3067 | 84 | 5'-CAP (32-51) | P=S |
| 3222 | 84 | 5'-CAP (32-51) | 2 ' - O - methyl; P=O |
| 3224 | 84 | 5'-CAP (32-51) | 2 ' - O - methyl; P=S |
| 3581 | 85 | 3'-Untranslated (1959-1978) | P=S |

Inhibition of ICAM-1 expression on the surface of interleukin-1β-stimulated cells by the oligonucleotides was determined by ELISA assay as described in Example 1. The oligonucleotides were tested in two different cell lines. None of the phosphodiester oligonucleotides inhibited ICAM-1 expression. This is probably due to the rapid degradation of phosphodiester oligonucleotides in cells. Of the five phosphorothioate oligonucleotides, the most active was ISIS 1570, which hybridizes to the AUG translation initiation codon. A 2'-o-methyl phosphorothioate oligonucleotide, ISIS 2974, was approximately threefold less effective than ISIS 1570 in inhibiting ICAM-1 expression in HUVEC and A549 cells. A 2'-fluoro oligonucleotide, ISIS 2634, was also less effective.

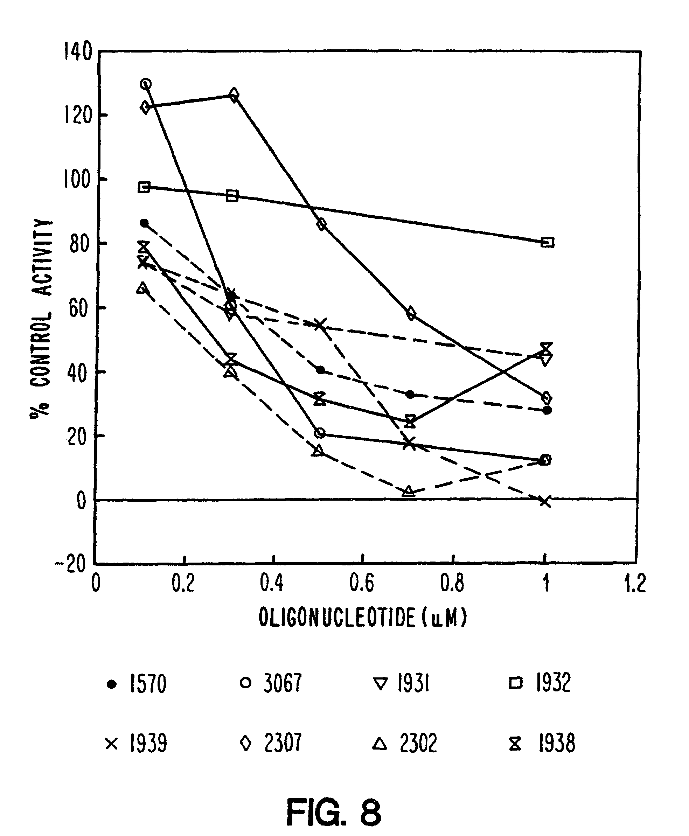

Based on the initial data obtained with the five original targets, additional oligonucleotides were designed which would hybridize with the ICAM-1 mRNA. The antisense oligonucleotide (ISIS 3067) which hybridizes to the predicted transcription initiation site (5' cap site) was approximately as active in IL-1β-stimulated cells as the oligonucleotide that hybridizes to the AUG codon (ISIS 1570), as shown in Figure 8. ISIS 1931 and 1932 hybridize 5' and 3', respectively, to the AUG translation initiation codon. All three oligonucleotides that hybridize to the AUG region inhibit ICAM-1 expression, though ISIS 1932 was slightly less active than ISIS 1570 and ISIS 1931. Oligonucleotides which hybridize to the coding region of ICAM-1 mRNA (ISIS 1933, 1934, 1935, 1574 and 1936) exhibited weak activity. Oligonucleotides that hybridize to the translation termination codon (ISIS 1937 and 1938) exhibited moderate activity.

Surprisingly, the most active antisense oligonucleotide was ISIS 1939, a phosphorothioate oligonucleotide targeted to a sequence in the 3'- untranslated region of ICAM-1 mRNA (see Table 1). Other oligonucleotides having the same sequence were tested, 2'-O-methyl (ISIS 2973) and 2'-fluoro (ISIS 2637); however, they did not exhibit this level of activity. Oligonucleotides targeted to other 3' untranslated sequences (ISIS 1572, 1573 and 1940) were also not as active as ISIS-1939. In fact, ISIS 1940, targeted to the polyadenylation signal, did not inhibit ICAM-1 expression.

Because ISIS 1939 proved unexpectedly to exhibit the greatest antisense activity of the original 16 oligonucleotides tested, other oligonucleotides were designed to hybridize to sequences in the 3'-untranslated region of ICAM-1 mRNA (ISIS 2302, 2303, 2304, 2305, and 2307, as shown in Table 1). ISIS 2307, which hybridizes to a site only five bases 3' to the ISIS 1939 target, was the least active of the series (Figure 8). ISIS 2302, which hybridizes to the ICAM-1 mRNA at a position 143 bases 3' to the ISIS 1939 target, was the most active of the series, with activity comparable to that of ISIS 1939. Examination of the predicted RNA secondary structure of the human ICAM-1 mRNA 3'-untranslated region (according to M. Zuker, *Science* **1989**, 244, 48-52) revealed that both ISIS 1939 and ISIS 2302 hybridize to sequences predicted to be in a stable stem-loop structure. Current dogma suggests that regions of RNA secondary structure should be avoided when designing antisense oligonucleotides. Thus, ISIS 1939 and ISIS 2302 would not have been predicted to inhibit ICAM-1 expression.

The control oligonucleotide ISIS 1821 did inhibit ICAM-1 expression in HUVEC cells with activity comparable to that of ISIS 1934; however, in A549 cells ISIS 1821 was less effective than ISIS 1934. The negative control, ISIS 1821, was found to have a small amount of activity against ICAM expression, probably due in part to its ability to hybridize (12 of 13 base match) to the ICAM-1 mRNA at a position 15 bases 3' to the AUG translation initiation codon.

These studies indicate that the AUG translation initiation codon and specific 3'-untranslated sequences in the ICAM-1 mRNA were the most susceptible to antisense oligonucleotide inhibition of ICAM-1 expression.

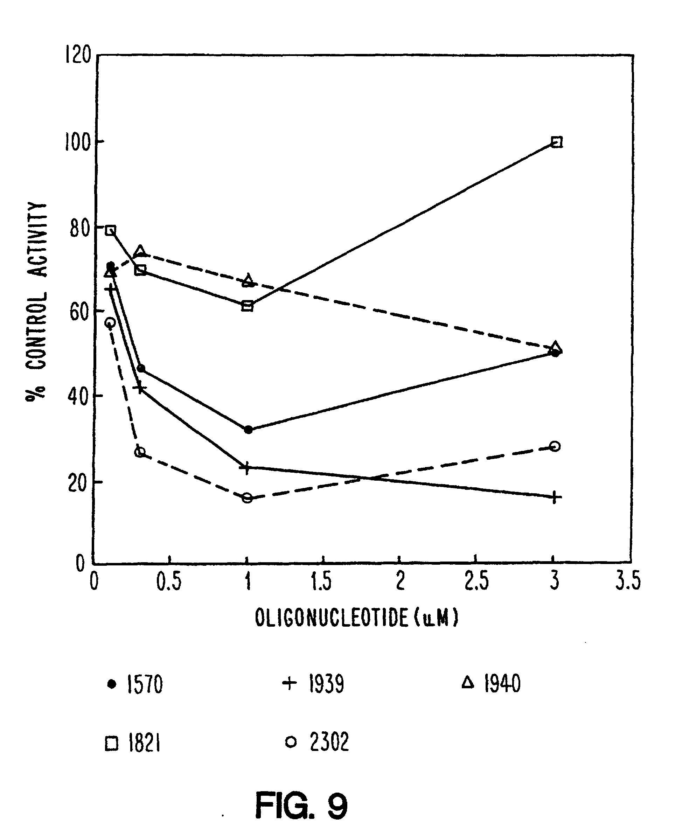

In addition to inhibiting ICAM-1 expression in human umbilical vein cells and the human lung carcinoma cells (A549), ISIS 1570, ISIS 1939 and ISIS 2302 were shown to inhibit ICAM-1 expression in the human epidermal carcinoma A431 cells and in primary human keratinocytes (shown in Figure 9). These data demonstrate that antisense oligonucleotides are capable of inhibiting ICAM-1 expression in several human cell lines. Furthermore, the rank order potency of the oligonucleotides is the same in the four cell lines examined. The fact that ICAM-1 expression could be inhibited in primary human keratinocytes is important because epidermal keratinocytes are an intended target of the antisense nucleotides.

### Example 6

### ELISA screening of antisense oligonucleotides for activity against ELAM-1 gene expression

Primary human umbilical vein endothelial (HUVEC) cells, passage 2 to 5, were plated in 96-well plates and allowed to reach confluence. Cells were washed three times with Opti-MEM (GIBCO, Grand Island NY). Cells were treated with increasing concentrations of oligonucleotide diluted in Opti-MEM containing 10 µg/ml DOTMA solution (Bethesda Research Labs, Bethesda MD) for 4 hours at 37°C. The medium was removed and replaced with EGM-UV (Clonetics, San Diego CA) plus oligonucleotide. Tumor necrosis factor α was added to the medium (2.5 ng/ml) and the cells were incubated an additional 4 hours at 37°C.

ELAM-1 expression was determined by ELISA. Cells were gently washed three times with Dulbecco's phosphate-buffered saline (D-PBS) prewarmed to 37°C. Cells were fixed with 95% ethanol at 4°C for 20 minutes, washed three times with D-PBS and blocked with 2% BSA in D-PBS. Cells were incubated with ELAM-1 monoclonal antibody BBA-1 (R&D Systems, Minneapolis MN) diluted to 0.5 µg/ml in D-PBS containing 2% BSA for 1 hour at 37°C. Cells were washed three times with D-PBS and the bound ELAM-1 antibody detected with biotinylated goat anti-mouse secondary antibody followed by β-galactosidase-conjugated streptavidin as described in Example 1.

The activity of antisense phosphorothioate oligonucleotides which target 11 different regions on the ELAM-1 cDNA and two oligonucleotides which target ICAM-1 (as controls) was determined using the ELAM-1 ELISA. The oligonucleotide and targets are shown in Table 2.

**TABLE 2**

| **ANTISENSE OLIGONUCLEOTIDES WHICH TARGET HUMAN ELAM-1** | | | |
|---|---|---|---|
| **ISIS NO.** | **SEQ ID NO.** | **TARGET REGION** | **MODIFICATION** |
| 1926 | 28 | AUG Codon (143-164) | P=S |
| 2670 | 29 | 3'-Untranslated (3718-3737) | P=S |
| 2673 | 30 | 3'-Untranslated (2657-2677) | P=S |
| 2674 | 31 | 3'-Untranslated (2617-2637) | P=S |
| 2678 | 32 | 3'-Untranslated (3558-3577) | P=S |
| 2679 | 33 | 5'-Untranslated (41-60) | P=S |
| 2680 | 34 | 3'-Untranslated (3715-3729) | P=S |
| 2683 | 35 | AUG Codon (143-163) | P=S |
| 2686 | 36 | AUG Codon (149-169) | P=S |
| 2687 | 37 | 5'-Untranslated (18-37) | P-S |
| 2693 | 38 | 3'-Untranslated (2760-2788) | P=S |
| 2694 | 39 | 3'-Untranslated (2934-2954) | P=S |

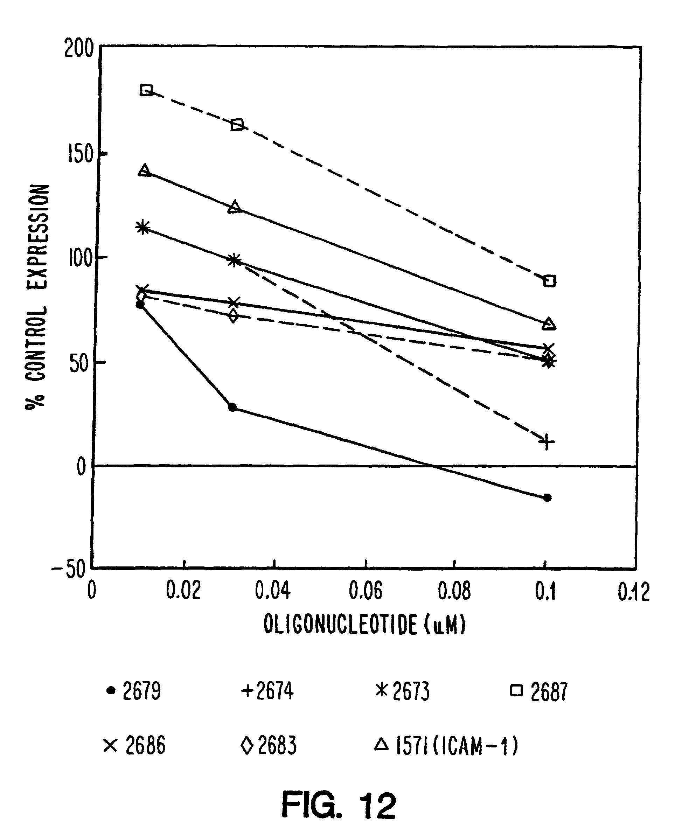

In contrast to what was observed with antisense oligonucleotides targeted to ICAM-1 (Example 5), the most potent oligonucleotide modulator of ELAM-1 activity (ISIS 2679) was hybridizable with specific sequences in the 5'-untranslated region of ELAM-1. ISIS 2687, an oligonucleotide which hybridized to sequences ending three bases upstream of the ISIS 2679 target, did not show significant activity (Figure 10). Therefore, ISIS 2679 hybridizes to a unique site on the ELAM-1 mRNA, which is uniquely sensitive to inhibition with antisense oligonucleotides. The sensitivity of this site to inhibition with antisense oligonucleotides was not predictable based upon RNA secondary structure predictions or information in the literature.

### Example 7

### ELISA screening of antisense oligonucleotides for activity against VCAM-1 gene expression

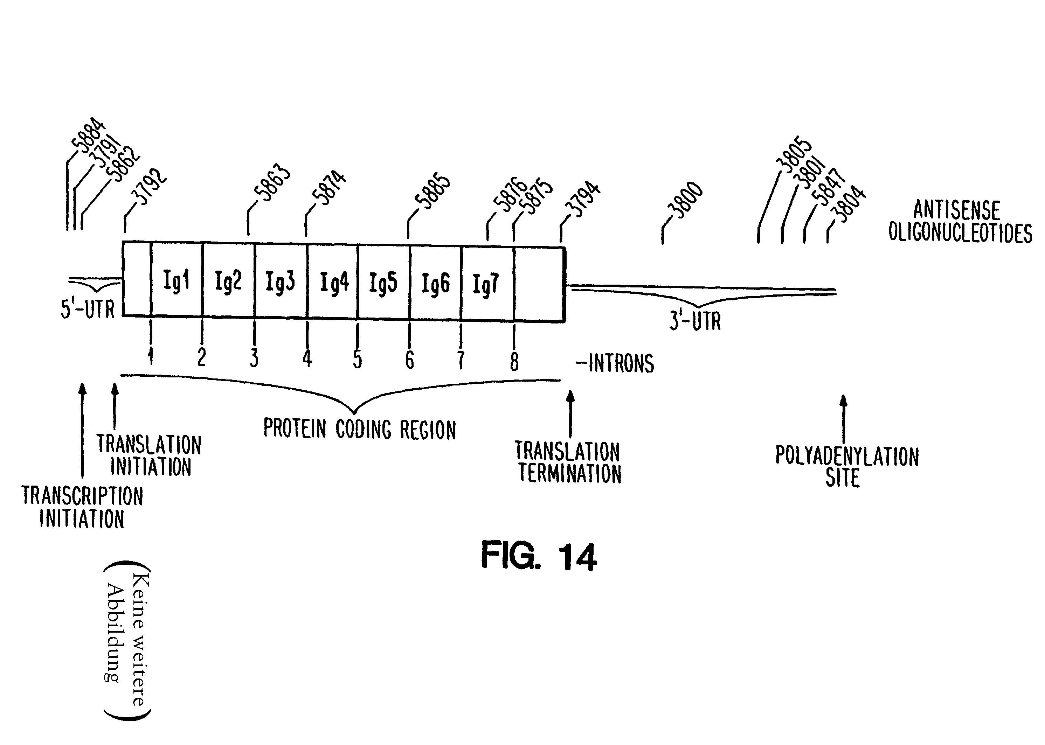

Inhibition of VCAM-1 expression by fifteen antisense phosphorothioate oligonucleotides was determined using the ELISA assay approximately as described in Representative Example 6, except that cells were stimulated with TNF-α for 16 hours and VCAM-1 expression was detected by a VCAM-1 specific monoclonal antibody (R & D Systems, Minneapolis, MN) used at 0.5 µg/ml. The target sites of these oligonucleotides on the VCAM-1 mRNA are shown in Figure 11. The sequence and activity of each oligonucleotide against VCAM-1 are shown in Table 3. The oligonucleotides indicated by an asterisk (*) have IC50's of approximately 50 nM or below and are preferred. IC50 indicates the dosage of oligonucleotide which results in 50% inhibition of VCAM-1 expression.

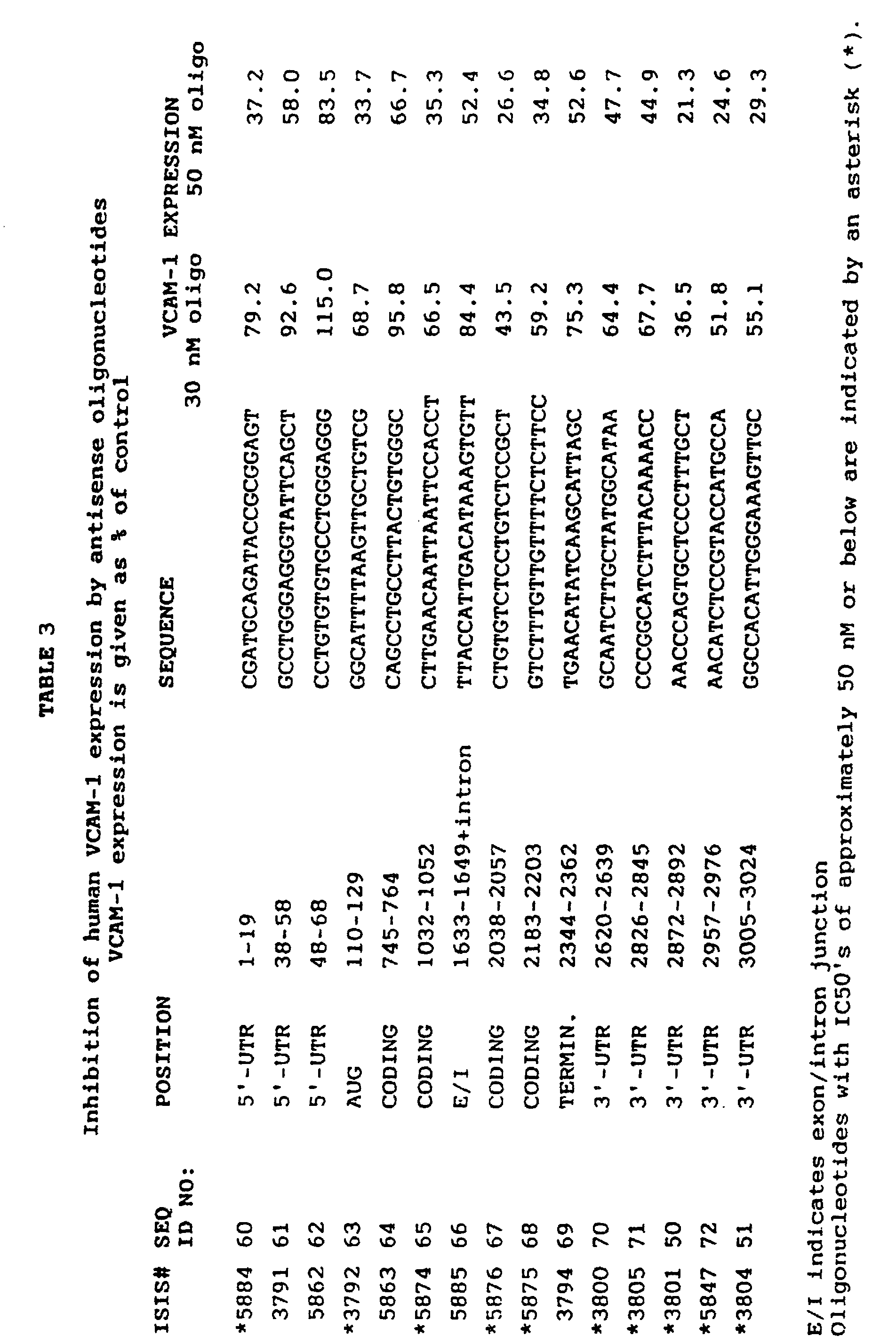

## Claims

1. An antisense oligonucleotide having up to 50 nucleotides which inhibits expression of vascular cell adhesion molecule-1, wherein said antisense oligonucleotide comprises the nucleotide sequence shown in SEQ ID NO: 67.

2. The antisense oligonucleotide of claims 1 wherein at least one of the linking groups between nucleotide units comprises a sulfur-containing species.

3. The antisense oligonucleotide of any one of claims 1 or 2 wherein at least one of the linking groups between nucleotide units comprises a phosphorothioate moiety.

4. A pharmaceutical composition comprising the antisense oligonucleotide of any one of claims 1 to 3 and a pharmaceutically acceptable carrier.

5. Use of an antisense oligonucleotide according to any one of claims 1 to 3 for the manufacture of a medicament for modulating synthesis of vascular cell adhesion molecule-1 in a cell or tissue.

6. An antisense oligonucleotide according to any one of claims 1 to 3 for use in treating an animal suspected of having a disease which is modulated by changes in vascular cell adhesion molecule-1.

7. The antisense oligonucleotide of claim 6 wherein the antisense oligonucleotide is administered topically.

8. The antisense oligonucleotide of claim 6 wherein the antisense oligonucleotide is administered systemically.

9. An antisense oligonucleotide according to any one of claims 1 to 3 for use in decreasing metastasis in an animal suspected of having a metastatic condition.

10. The antisense oligonucleotide of claim 9 wherein the metastastic is in the lung.

11. The antisense oligonucleotide of any one of the claims 9 or 10 wherein the antisense oligonucleotide is administered in a pharmaceutical composition comprising the antisense oligonucleotide and a pharmaceutically acceptable carrier.

12. A ex vivo method for modulating synthesis of vascular cell adhesion molecule-1 in a cell or tissue comprising contacting said cell or tissue with an antisense oligonucleotide according to any one of claims 1 to 3.

## Patentansprüche

1. Antisense-Oligonukleotid mit bis zu 50 Nukleotiden, welches die Expression des vaskulären Zelladhäsionsmoleküls-1 (VCAM-1) inhibiert, wobei das Antisense-Oligonukleotid die in SEQ ID Nr. 67 gezeigte Nukleotidsequenz umfaßt.

2. Antisense-Oligonukleotid nach Anspruch 1, wobei mindestens eine der Verbindungsgruppen zwischen den Nukleotideinheiten eine Schwefelenthaltende Spezies umfaßt.

3. Antisense-Oligonukleotid nach einem der Ansprüche 1 oder 2, wobei mindestens eine der Verbindungsgruppen zwischen den Nukleotideinheiten eine Phosphorthioatkomponente umfaßt.

4. Pharmazeutische Zusammensetzung, die das Antisense-Oligonukleotid nach einem der Ansprüche 1 bis 3 und einen pharmazeutisch verträgtichen Träger umfaßt.

5. Verwendung eines Antisense-Oligonukleotids nach einem der Ansprüche 1 bis 3 für die Herstellung eines Medikaments zur Modulation der Synthese des vaskulären Zelladhäsionsmoleküls-1 in einer Zelle oder einem Gewebe.

6. Antlsense-Oligonukleotid nach einem der Ansprüche 1 bis 3 zur Verwendung bei der Behandlung eines Tieres mit Verdacht auf eine Krankheit, die durch Änderungen im vaskulären Zelladhäsionsmolekül-1 moduliert ist.

7. Antisense-Oligonukleotid nach Anspruch 6, wobei das Antisense-Oligonukleotid topisch verabreicht wird.

8. Antisense-Oligonukleotid nach Anspruch 6, wobei das Antisense-Oligonukleotid systemisch verabreicht wird.

9. Antisense-Oligonukleotid nach einem der Ansprüche 1 bis 3 zur Verwendung bei der Verringerung der Metastasierung in einem Tier mit Verdacht auf einen metastasenbildenden Zustand.

10. Antisense-Oligonukleotid nach Anspruch 9, wobei die Metastasenbildung in der Lunge ist.

11. Antisense-Oligonukleotid nach einem der Ansprüche 9 oder 10, wobei das Antisense-Oligonukleotid in einer pharmazeutischen Zusammensetzung verabreicht wird, die das Antisense-Oligonukleotid und einen pharmazeutisch verträglichen Träger umfaßt.

12. *Ex vivo*-Verfahren zur Modulation der Synthese des vaskulären Zelladnäsionsmoleküls-1 in einer Zelle oder einem Gewebe, umfassend das Inkontaktbringen der Zelle oder des Gewebes mit einem Antisense-Oligonukleotid nach einem der Ansprüche 1 bis 3.

## Revendications

1. Un oligonucléotide antisens ayant jusqu'à 50 nucléotides, qui inhibe la molécule d'adhésion cellulaire vasculaire 1, dans laquelle cet oligonucléotide antisens comporte la séquence nucléotidique décrite dans le NO 67 ID SEQ.

2. L'oligonucléotide antisens de la revendication 1 dans laquelle l'un au moins des groupes de liaison entre les unités nucléotidiques est constitué d'une espèce soufrée.

3. L'oligonucléotide antisens de l'une quelconque des revendications 1 ou 2, dans laquelle l'un au moins des groupes de liaison entre les unités nucléotidiques est constitué d'un fragment phosphorothioate.

4. Une composition pharmaceutique comprenant l'oligonucléotide antisens de l'une quelconque des revendications 1 à 3 et un véhicule acceptable pharmaceutiquement.

5. Utilisation d'un oligonucléotide antisens selon l'une quelconque des revendications 1 à 3 pour la fabrication d'un médicament destiné à moduler la synthèse de molécule d'adhésion cellulaire vasculaire 1 dans une cellule ou un tissu.

6. Un oligonucléotide antisens selon l'une quelconque des revendications 1 à 3 destiné à être utilisé pour le traitement d'un animal dont on suspecte qu'il a une maladie qui est modulée par des modifications de la molécule d'adhésion cellulaire vasculaire 1.

7. L'oligonucléotide antisens de la revendication 6 dans laquelle l'oligonucléotide antisens est administré de manière topique.

8. L'oligonucléotide antisens de la revendication 6 dans laquelle l'oligonucléotide antisens est administré de manière systémique.

9. Un oligonucléotide antisens selon l'une quelconque des revendications 1 à 3 destiné à être utilisé pour diminuer les métastases chez un animal dont on suspecte qu'il a une pathologie à métastases.

10. L'oligonucléotide antisens de la revendication 9 dans laquelle la métastase est située dans le poumon.

11. L'oligonucléotide antisens de l'une quelconque des revendications 9 ou 10 dans lesquelles l'oligonucléotide antisens est administré dans une composition pharmaceutique comprenant l'oligonucléotide antisens et un véhicule acceptable pharmaceutiquement.

12. Un procédé *ex vivo* pour moduler la synthèse de la molécule d'adhésion cellulaire vasculaire 1 dans une cellule ou un tissu qui consiste en la mise en contact de cette cellule ou de ce tissu avec un oligonucléotide antisens selon l'une quelconque des revendications 1 à 3.
